# EUROPÄISCHE PATENTSCHRIFT

(11) **EP 0 846 113 B1**
(45) Veröffentlichungstag und Bekanntmachung des Hinweises auf die Patenterteilung: **29.01.2003**
(21) Anmeldenummer: 96927702.9
(22) Anmeldetag: 07.08.1996
(51) Int. Cl.: C07D 401/04, A01N 43/56, A01N 43/40

(54) **1-(PYRIDYL)-PYRAZOLE UND IHRE VERWENDUNG ALS HERBIZIDE**
1-(PYRIDYL)-PYRAZOLS AND THEIR USE AS HERBICIDES
1-(PYRIDYL)-PYRAZOLES ET LEUR UTILISATION COMME HERBICIDES

(30) Priorität: 21.08.1995 DE 19530606
(43) Veröffentlichungstag der Anmeldung: 10.06.1998
(73) Patentinhaber: BASF AKTIENGESELLSCHAFT, 67056 Ludwigshafen (DE)
(72) Erfinder: HAMPRECHT, Gerhard, D-69469 Weinheim (DE); HEISTRACHER, Elisabeth, D-67061 Ludwigshafen (DE); KLINTZ, Ralf, D-67269 Gruenstadt (DE); SCHÄFER, Peter, D-67308 Ottersheim (DE); ZAGAR, Cyrill, D-67061 Ludwigshafen (DE); SCHIFFER, Helmut, D-67112 Mutterstadt (DE); WESTPHALEN, Karl-Otto, D-67346 Speyer (DE); MISSLITZ, Ulf, D-67433 Neustadt (DE); WALTER, Helmut, D-67283 Obrigheim (DE)
(86) Internationale Anmeldenummer: EP9603493
(87) Internationale Veröffentlichungsnummer: WO97007114

(56) Entgegenhaltungen:
- EP-A- 0 204 242
- EP-A- 0 205 021
- EP-A- 0 207 285
- US-A- 5 167 691

## Beschreibung

Die vorliegende Erfindung betrifft neue 1-(Pyridyl)-pyrazole, Verfahren zu ihrer Herstellung und ihre Verwendung als Herbizide.

Es ist bereits bekannt, daß bestimmte 1-(Pyridyl)-pyrazole, z. B. das 4-Cyano-5-amino-1-(3-chlor-5-trifluormethyl-pyridyl-2)-pyrazol (DE-OS 3408 727), das 4-Nitro-5-amino-1-(3-chlor-5-trifluormethyl-pyridyl-2)-pyrazol, das 4-Ethoxycarbonyl-5-amino-1-(3-chlor-5-trifluormethyl-pyridyl-2)-pyrazol (DE-OS 3520 330), dort beschriebene weitere Derivate, das 4-Cyano-5-n-propylamino-1-(3,5-dichlor-pyridyl-2)-pyrazol (DE 3520 327), das 4-Chlor-5-methylsulfonylamino-1-(3-chlor-5-trifluormethyl-pyridyl-2)-pyrazol (JP 2142 785), das 5-Amino-1-(3,6-dichlor-5-trifluormethyl-2-pyridyl)-4-cyano-pyrazol (US 5,167,691) sowie das 5-Cyano-1-(pyridyl-2)-N-methylpyrazol-4-carboxamid (US 4589 905) herbizide Eigenschaften haben. Die EP-A 0 204 242 und die EP-A 0 205 021 beschreiben 1-Pyridyl-4-cyano-5-halogenpyrazole mit herbiziden Eigenschaften, bei denen der Pyridyl-Rest zweifach substituiert ist.

Das 5-Amino-3-cyano-4-trifluormethylsulfonyl-1-(3-chlor-5-trifluormethylpyridyl-2)-pyrazol besitzt insektizide Eigenschaften (EP 500 209).

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen.

USP 5198 014 beschreibt die 3-(4-Cyano-1-(3-chlor-5-trifluormethyl-pyridyl-2)-pyrazolyl-5)-acrylsäure (Beispiel 4, Verbindung Nr. 14, Tabelle 3) und die entsprechend in der Pyridin-6-Position substituierte Chlorverbindung (Nr. 19, Tabelle 3). Wie der Vergleich der herbiziden Wirkung zeigt, wirkt die Pyridin-6-H-Verbindung 14 wesentlich stärker herbizid als die Pyridin-6-Cl-Verbindung 19.

Überraschend wurde nun gefunden, daß neue substituierte 1-(Pyridyl)-pyrazole der allgemeinen Formel I 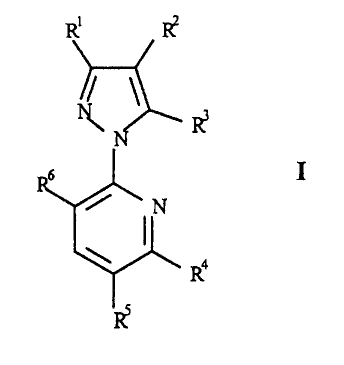 in der die Substituenten die folgende Bedeutung haben:
- R¹: Wasserstoff;
- R²: C₁-C₄-Alkoxy, C₁-C₄-Halogenalkoxy, NO₂, Halogen, Rhodano, Amino, C(=O)R⁷, S(O)ₙR⁸ oder NH-C(=O)R⁹;
- R³: Amino, Halogen, Rhodano, Cyano, Nitro, Hydroxy, C₁-C₃-Alkyl, C₁-C₃-Halogenalkyl, S(O)ₙR⁸, N(R¹⁰)R¹¹, OR¹², SR¹³, N=C(R¹⁴)-N(R¹⁵)R¹⁶ oder N=C(R¹⁷)R¹⁸;
- R⁴: Halogen, einen Rest XR¹⁹;
- R⁵ und R⁶: unabhängig voneinander Halogen oder Trifluormethyl;
- R⁷: C₁-C₄-Alkyl, C₁-C₄-Halogenalkyl, C₁-C₄-Alkoxy oder C₁-C₄-Alkylamino;
- R⁸: C₁-C₄-Alkyl, Trifluormethyl, C₁-C₄-Alkoxy, Chlor, Amino, Methylaminooder Ethylamino;
- R⁹: C₁-C₄-Alkyl, C₁-C₄-Halogenalkyl oder C₁-C₄-Alkoxy;
- R¹⁰: Wasserstoff, C₁-C₄-Alkyl oder C(=O)R⁷;
- R¹¹: C₁-C₄-Alkyl, C₃-C₄-Alkenyl, C₃-C₄-Alkinyl, C(=O)R⁷ oder S(O)ₙR⁸;
- R¹²: C₁-C₃-Alkyl, C₁-C₃-Halogenalkyl, C₃-C₄-Alkenyl, C₃-C₄-Alkinyl oder C₁-C₅-Alkoxycarbonyl-C₁-C₄-alkyl;
- R¹³: C₁-C₄-Alkoxycarbonyl-C₁-C₄-alkyl;
- R¹⁴,¹⁶,¹⁷: Wasserstoff oder C₁-C₃-Alkyl;
- R¹⁵: C₁-C₄-Alkyl;
- R¹⁸: C₁-C₄-Alkyl, C₁-C₄-Halogenalkyl oder C₂-C₄-Alkenyl;
- R¹⁹: C₁-C₄-Alkyl, C₃-C₄-Alkenyl, C₃-C₄-Alkinyl, C₁-C₄-Halogenalkyl, C₁-C₄-Cyanoalkyl, C₁-C₄-Alkoxycarbonyl-C₁-C₄-alkyl, im Falle von X = O zusätzlich C₁-C₄-Alkylamino oder C₁-C₄-Alkylidenimino;
- X: Sauerstoff oder Schwefel;

Halogen Fluor oder Chlor und
- n: 0, 1 oder 2 bedeuten,
sowie die N-Oxide und die landwirtschaftlich brauchbaren Salze der Verbindungen I, eine gute herbizide Wirkung besitzen und verträglich in grasartigen Kulturen wie Weizen oder Reis sind.

Außerdem betrifft die Erfindung herbizide Mittel, welche die Verbindungen I als wirksame Substanzen enthalten, sowie Verfahren zur Herstellung der Verbindungen I und von herbiziden Mitteln unter Verwendung der Verbindungen I.

Die vorstehend für die Substituenten R¹ bis R¹⁹ in der Formel I genannten Bedeutungen stellen Sammelbegriffe für eine individuelle Aufzählung der einzelnen Gruppenmitglieder dar. Sämtliche Kohlenstoffketten, also alle Alkyl-, Alkenyl-, Alkinyl-, Halogenalkyl- und Halogenalkoxyteile, können geradkettig oder verzweigt sein. Halogenierte Substftuenten tragen vorzugsweise 1 - 6 gleiche oder verschiedene Halogenatome.

Im einzelnen bedeuten beispielsweise:
- Halogen: Fluor, Chlor, Brom und Jod, vorzugsweise Fluor und Chlor;
- C₁-C₄-Alkyl: Methyl, Ethyl, n-Propyl, 1-Methylethyl, n-Butyl, 1-Methylpropyl, 2-Methylpropyl und 1,1-Dimethylethyl;
- C₁-C₆-Alkyl: C₁-C₄-Alkyl wie vorstehend genannt, sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
- C₂-C₆-Alkenyl: Ethenyl, Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methylprop-2-en-1-yl und 2-Methyl-prop-2-en-1-yl, n-Penten-1-yl, n-Penten-2-yl, n-Penten-3-yl, n-Penten-4-yl, 1-Methylbut-1-en-1-yl, 2-Methyl-but-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methyl-but-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methylpent-1-en-1-yl, 3-Methyl-pent-1-en-1-yl, 4-Methylpent-1-en-1-yl, 1-Methyl-pent-2-en-1-yl, 2-Methylpent-2-en-1-yl, 3-Methyl-pent-2-en-1-yl, 4-Methylpent-2-en-1-yl, 1-Methyl-pent-3-en-1-yl, 2-Methylpent-3-en-1-yl, 3-Methyl-pent-3-en-1-yl, 4-Methylpent-3-en-1-yl, 1-Methyl-pent-4-en-1-yl, 2-Methylpent-4-en-1-yl, 3-Methyl-pent-4-en-1-yl, 4-Methylpent-4-en-1-yl, 1,1-Dimethyl-but-2-en-1-yl, 1,1-Dimethylbut-3-en-1-yl, 1,2-Dimethyl-but-l-en-1-yl, 1,2-Dimethylbut-2-en-1-yl, 1,2-Dimethyl-but-3-en-1-yl, 1,3-Diemthylbut-1-en-1-yl, 1,3-Dimethyl-but-2-en-1-yl, 1,3-Dimethylbut-3-en-1-yl, 2,2-Dimethyl-but-3-en-1-yl, 2,3-Dimethylbut-1-en-1-yl, 2,3-Dimethyl-but-2-en-1-yl, 2,3-Dimethylbut-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethylbut-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methylprop-2-en-1-yl, vorzugsweise Ethenyl und Prop-2-en-1-yl;
- C₂-C₆-Alkinyl: Ethinyl und C₃-C₆-Alkinyl wie Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methylpent-1-in-5-yl, 4-Methyl-pent-1-in-1-yl, 4-Methylpent-2-in-4-yl und 4-Methyl-pent-2-in-5-yl, vorzugsweise Prop-2-in-1-yl, 1-Methyl-prop-2-in-1-yl;
- C₁-C₄-Halogenalkyl; C₁-C₄-Alkyl wir vorstehend genannt, das partiell oder vollständig durch Fluor, Chlor und / oder Brom substituiert ist, also z. B., Chlormethyl, Dichlormethyl, Trichlormethyl, Fluormethyl, Difluromethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chloropropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl oder 4-Brombutyl;
- Cyano-(C₁-C₆)-alkyl: C₁-C₆-Alkyl wie vorstehend genannt, wobei jeweils ein Wasserstoffatom durch die Cyanogruppe ersetzt ist, also z. B. Cyanomethyl, 1-Cyanoeth-1-yl, 2-Cyanoeth-1-yl, 1-Cyano-prop-1-yl, 2-Cyano-prop-1-yl, 3-Cyanoprop-1-yl, 1-Cyano-prop-2-yl, 2-Cyano-prop-2-yl, 1-Cyanobut-1-yl, 2-Cyano-but-1-yl, 3-Cyano-but-1-yl, 4-Cyanobut-1-yl, 1-Cyano-but-2-yl, 2-Cyano-but-2-yl, 1-Cyanobut-3-yl, 2-Cyano-but-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl, vorzugsweise Cyanomethyl, 1-Cyano-1-methyl-ethyl;
- C₁-C₄-Alkoxy: Methoxy, Ethoxy, n-Propoxy, 1-Methylethoxy, n-Butoxy, 1-Methylpropoxy, 2-Methylpropoxy oder 1,1-Dimethylethoxy;
- C₁-C₆-Alkoxy für: C₁-C₄-Alkoxy wie vorstehend genannt, sowie für n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy:
- C₁-C₃-Halogenalkoxy für: C₁-C₃-Alkoxy wie vorstehend genannt, das partiell oder vollständig durch Fluor, Chlor. Brom und /oder Jod substituiert ist, also z. B. Difluromethoxy, Trifluormethoxy, Chlordifluormethoxy, Bromdifluromethoxy, 2-Fluorethoxy, 2-Chlorethoxy, 2-Bromethoxy, 2-Jodethoxy, 2,2-Difluroethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluropropoxy, 2,3-Difluropropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1- (Fluormethyl)-2-fluorethoxy, 1-(Chlormethyl)-2-chlorethoxy oder 1-(Brommethyl)-2-bromethoxy;
- C₂-C₄-Alkenyloxy: Eth-1-en-1-yloxy, Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy;
- C₃-C₄-Alkinyloxy: Prop-1-in-1-yloxy, Prop-2-in-1-yloxy, n-But-1-in-1-yloxy, n-But-1-in-3-yloxy, n-But-1-in-4-yloxy, n-But-2-in-4-yloxy;
- C₃-C₆-Cycloalkyl: Cyclopropyl, Cyclobutyl, Cyclopentyl oder Cyclohexyl;
- C₁-C₄-Alkylamino: Methylamino, Ethylamino, n-Propylamino, 1-Methylethylamino, n-Butylamino, 1-Methylpropylamino, 2-Methylpropylamino und 1,1-Dimethylethylamino, vorzugsweise Methylamino und Ethylamino;
- Di-(C₁-C₄-alkyl)amino: N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl) amino, N-Methyl-N-(2-methylpropyl)amino, N-(1,1-Dimethylethyl-N-methylamino, N-Ethyl-N-propylamino, N-Ethyl-N-(1-methylethyl)amino, N-Butyl-N-ethylamino, N-Ethyl-N-(1-methyl-propyl)amino, N-Ethyl-N-(2-methyl-propyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methyl-propyl)amino, N-(1,1-Dimethylethyl)-N-(1-methylethyl)amino, N-Butyl-N-(1-methylpropyl)amino, N-Butyl-N-(2-methyl-propyl)amino, N-Butyl-N-(1,1-dimethylethyl)amino, N-(1-Methylpropyl)-N-(2-methylpropyl)amino, N-(1,1-Dimetyhlethyl)-N-(1-methylpropyl)amino und N-(1,1-Dimethylethyl)-N-(2-methylpropyl)amino, vorzugsweise Dimethylamino und Diethylamino;
- C₁-C₆-Alkylidenimino: Ethylidenimino, Propylidenimino, Butylidenimino, 2-Methyl-propylidenimino, Pentylidenimino, 2-Methyl-butylidenimino, 3-Methyl-butylidenimino, Hexylidenimino, 2-Methyl-pentylidenimino, 3-Methylpentylidenimino oder 2-Ethylbutylidenimino.

Besonders bevorzugt sind die in den folgenden Tabellen 1-8 aufgeführten Verbindungen:

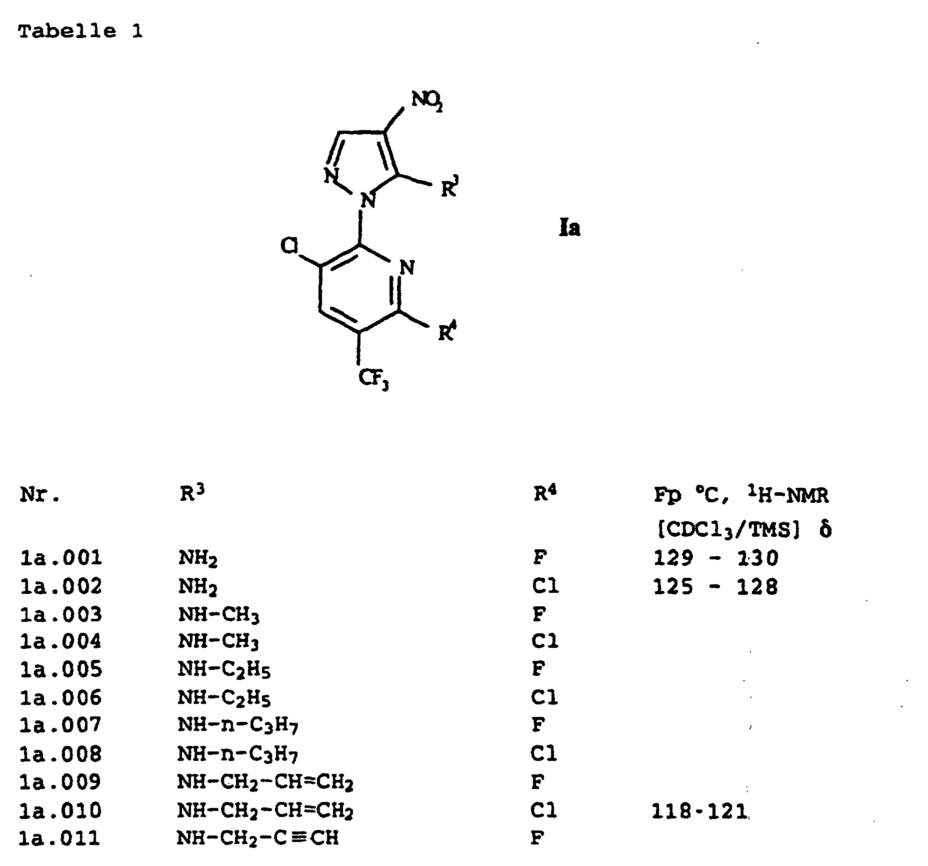

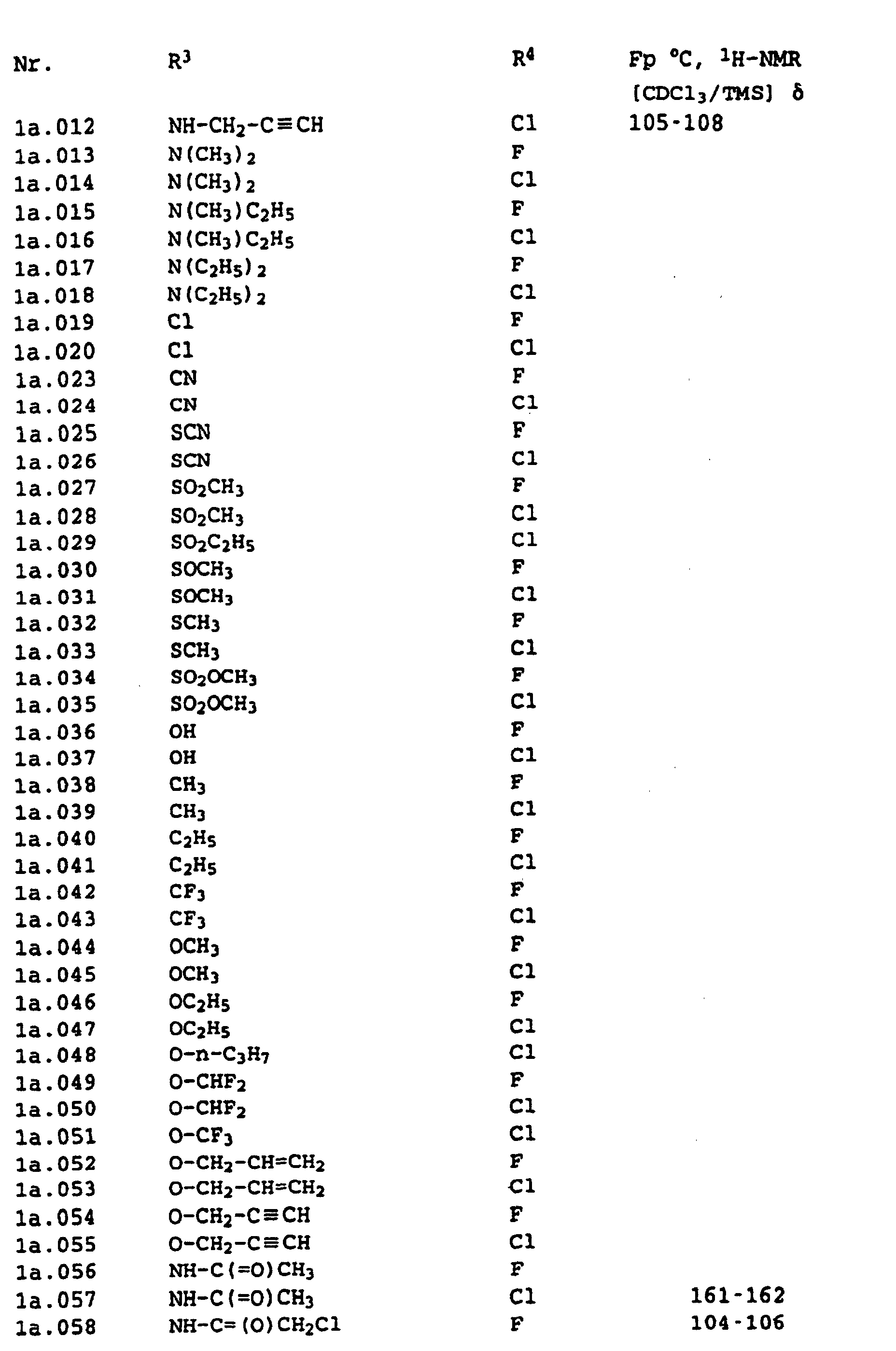

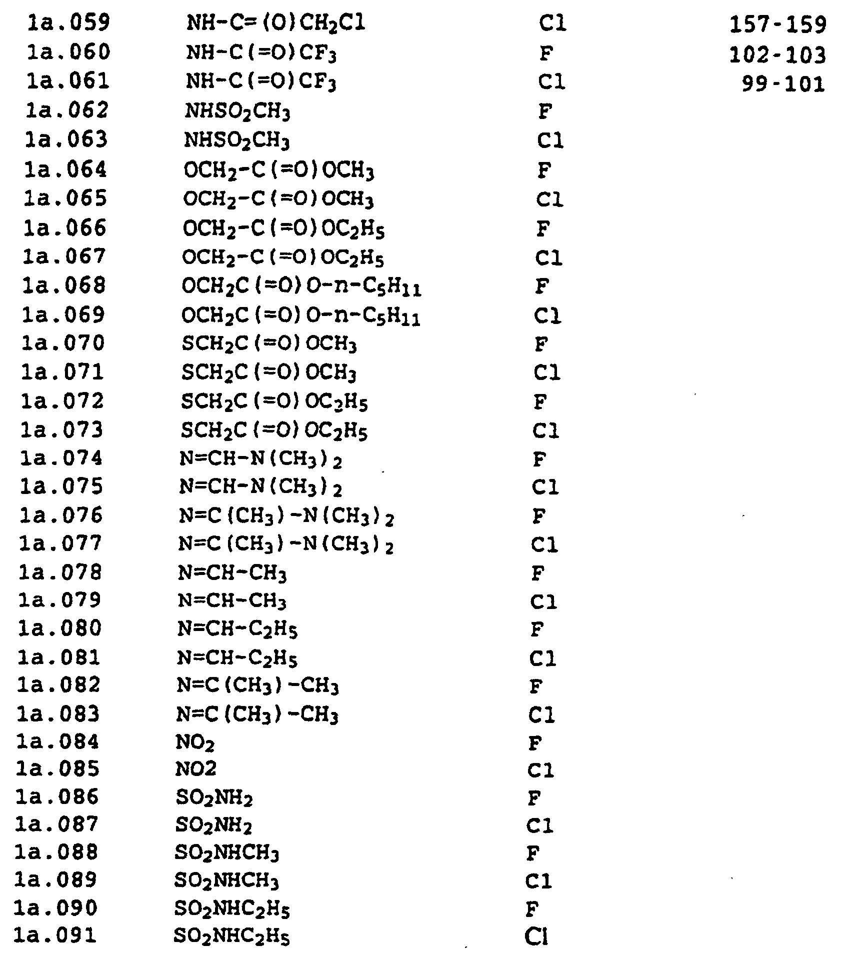

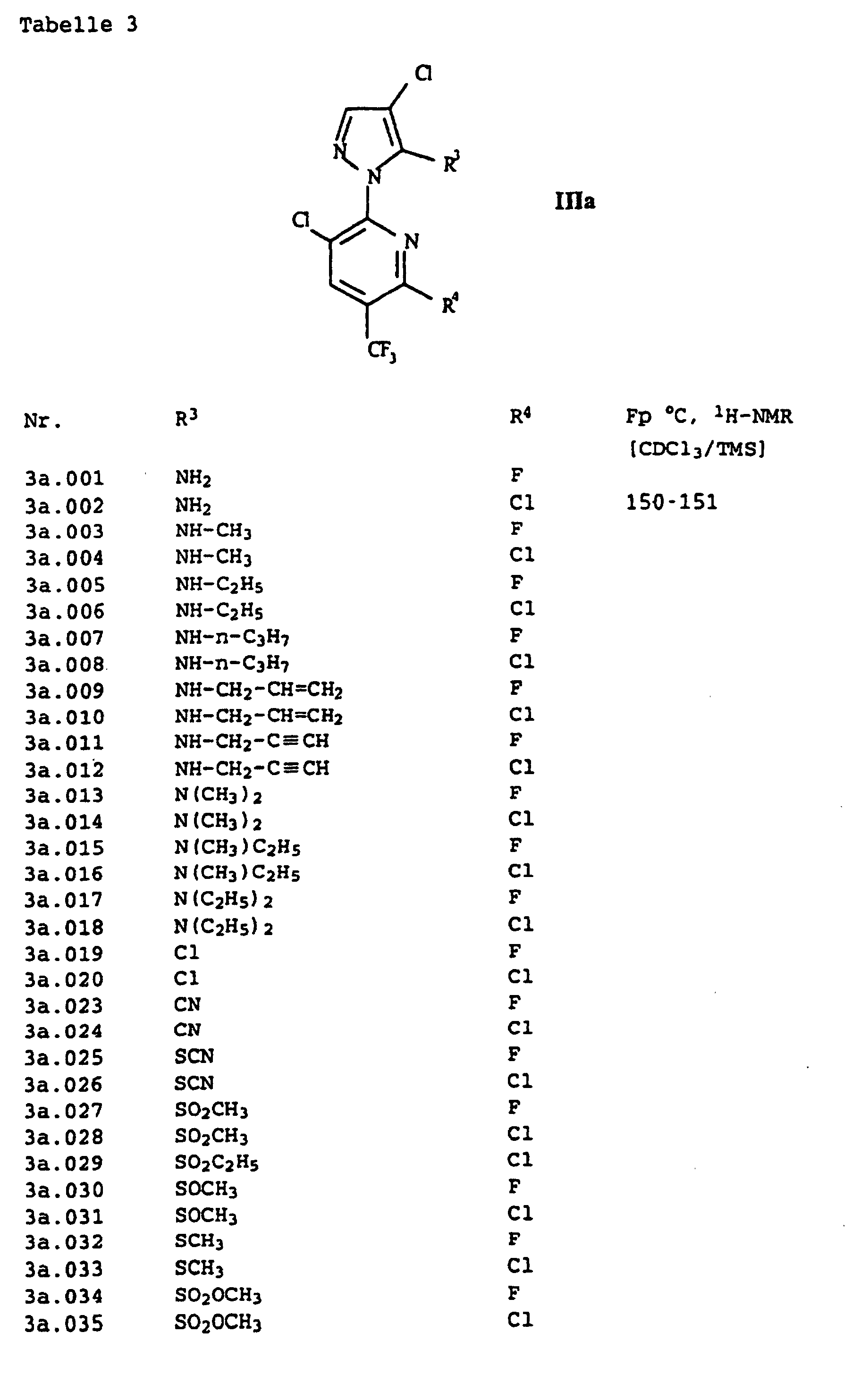

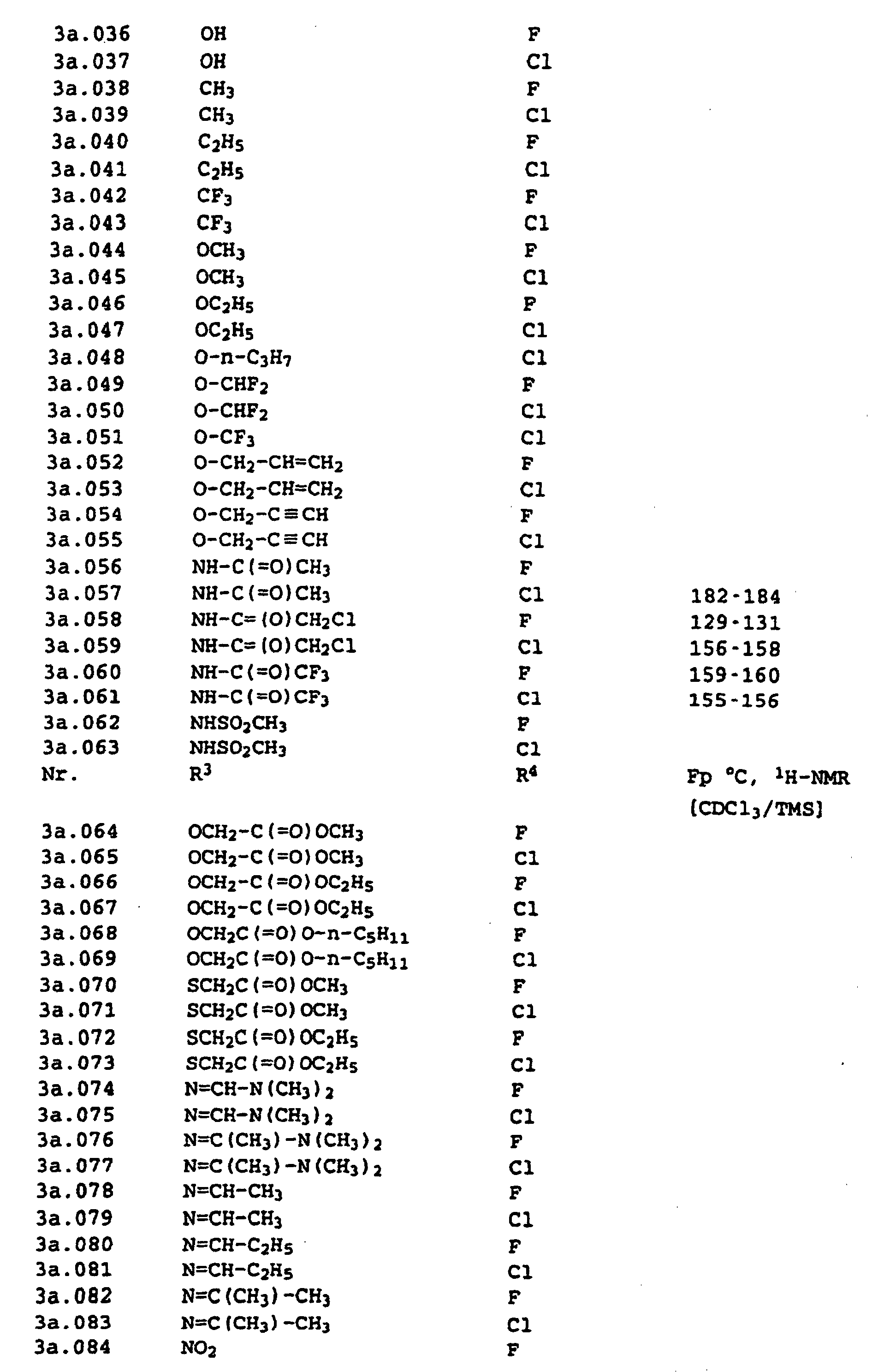

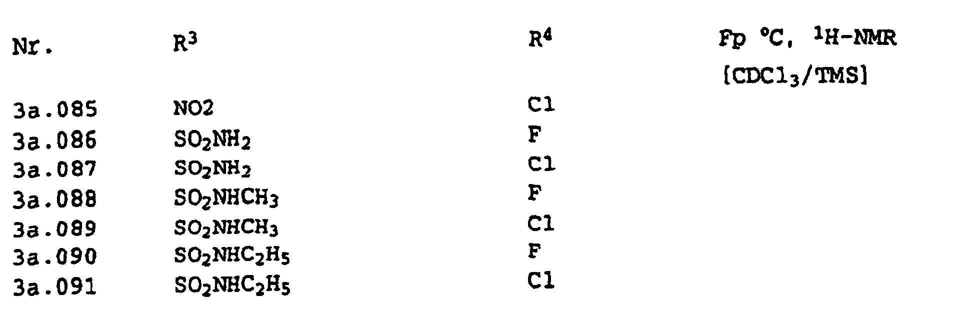

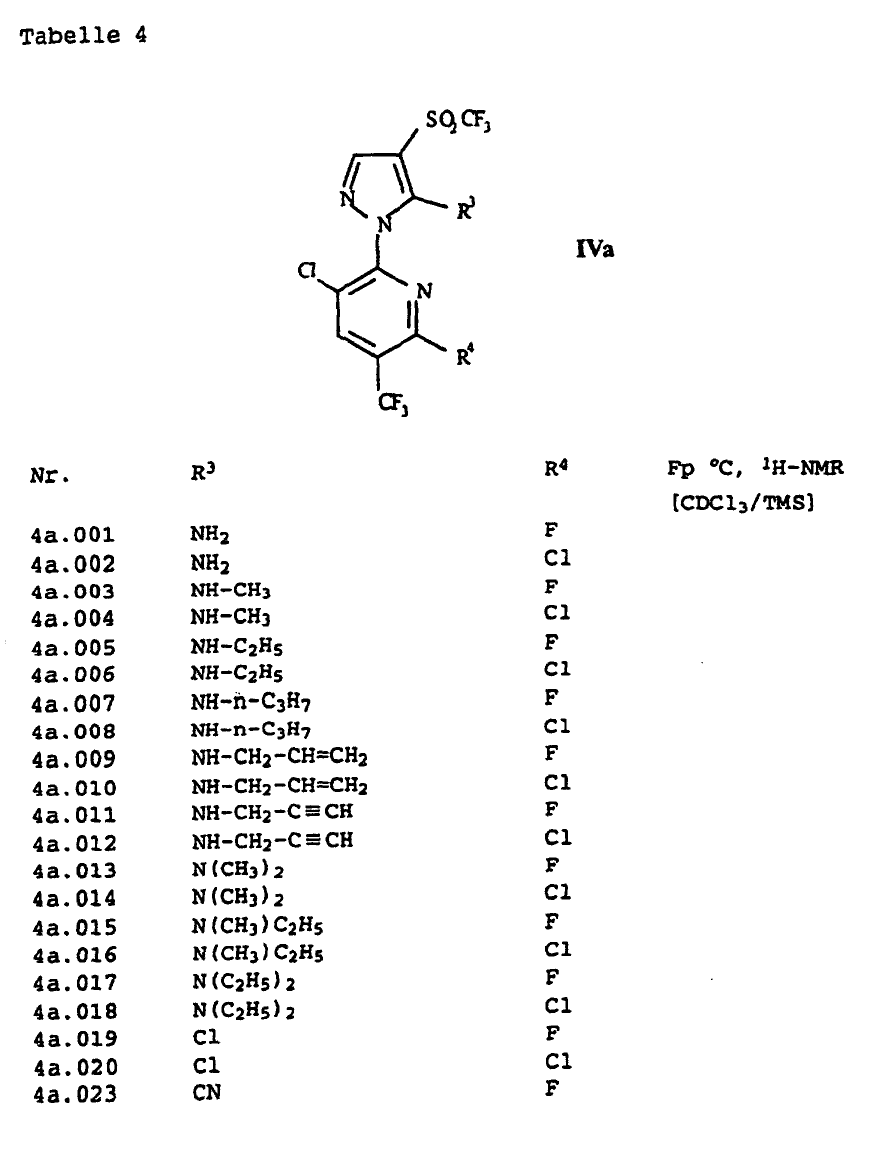

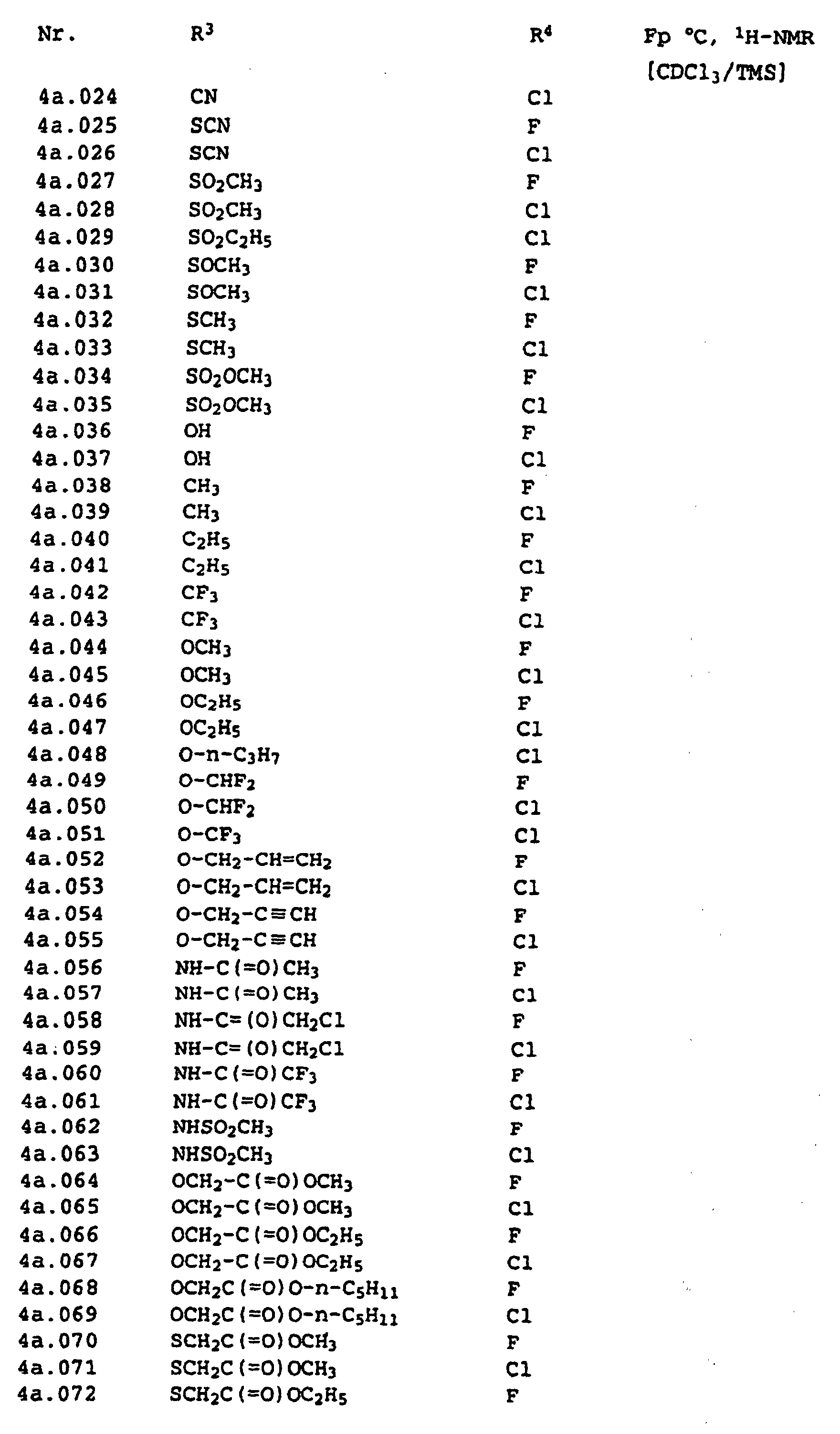

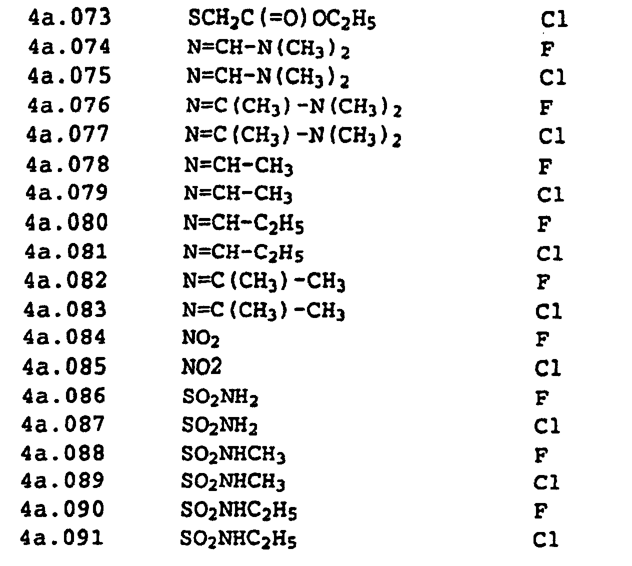

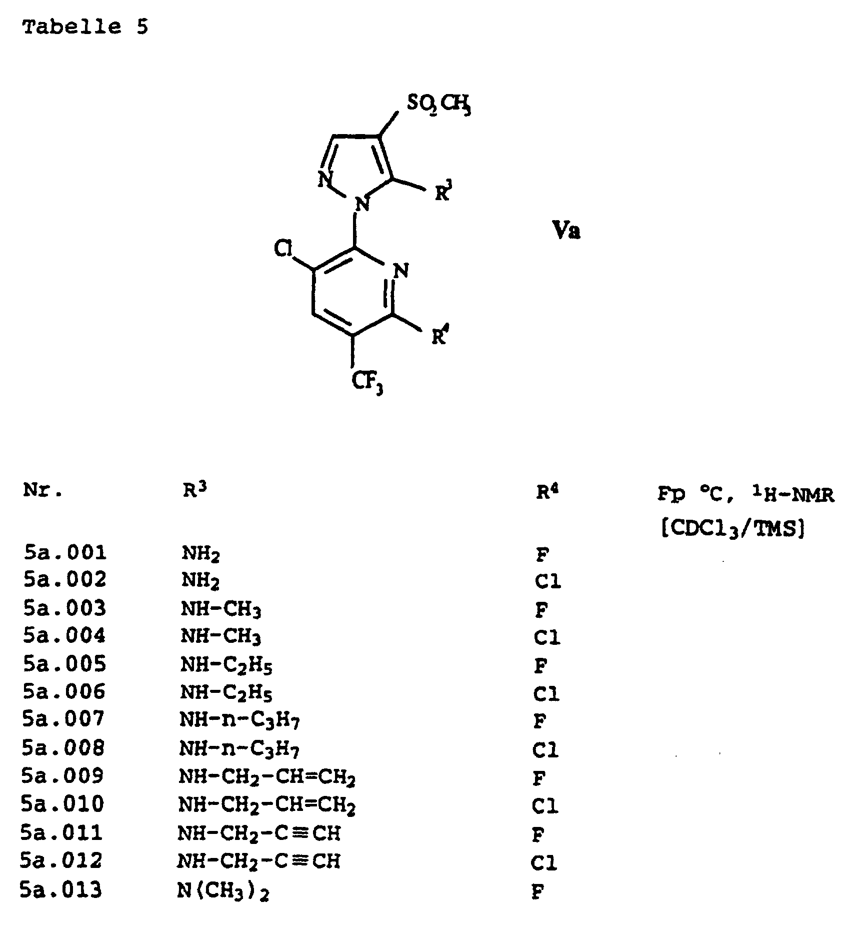

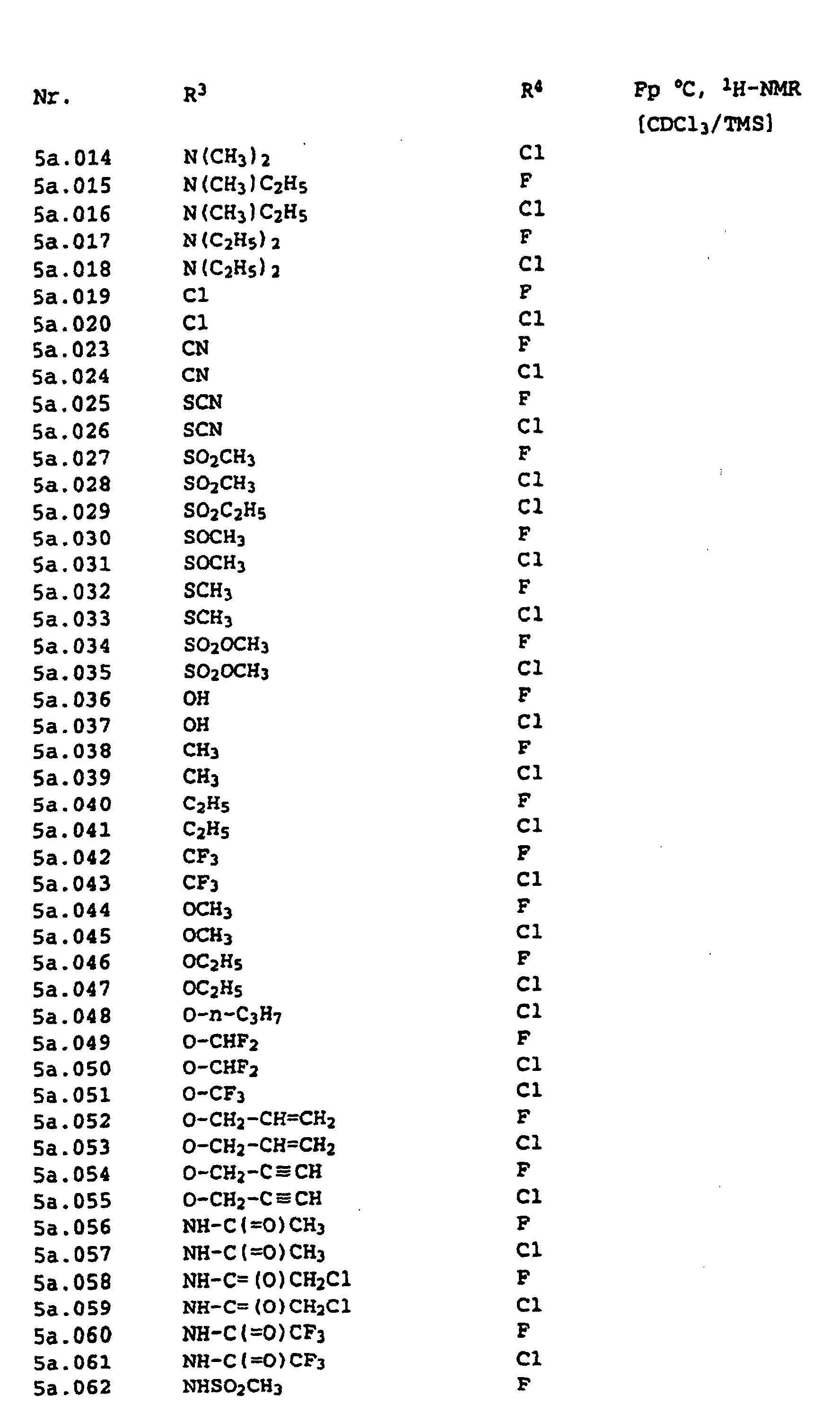

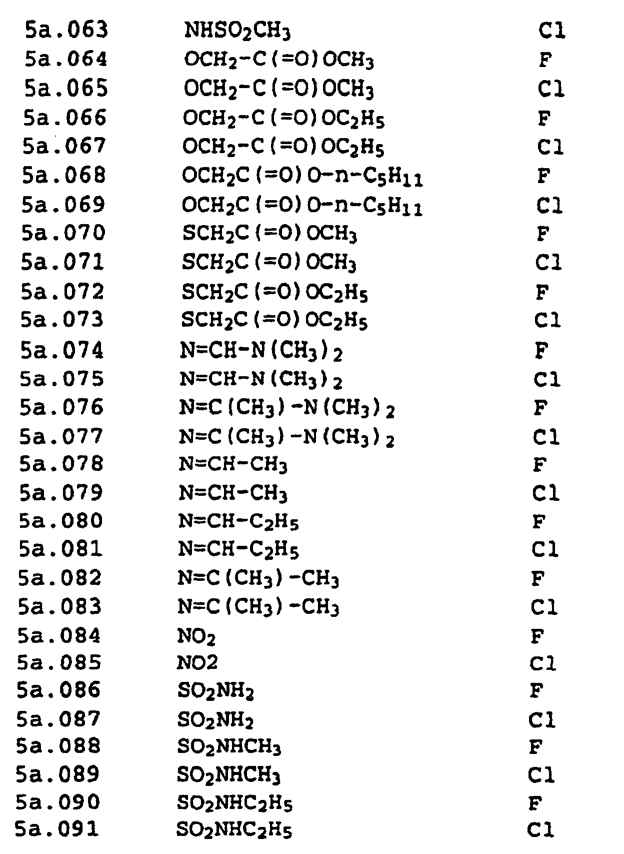

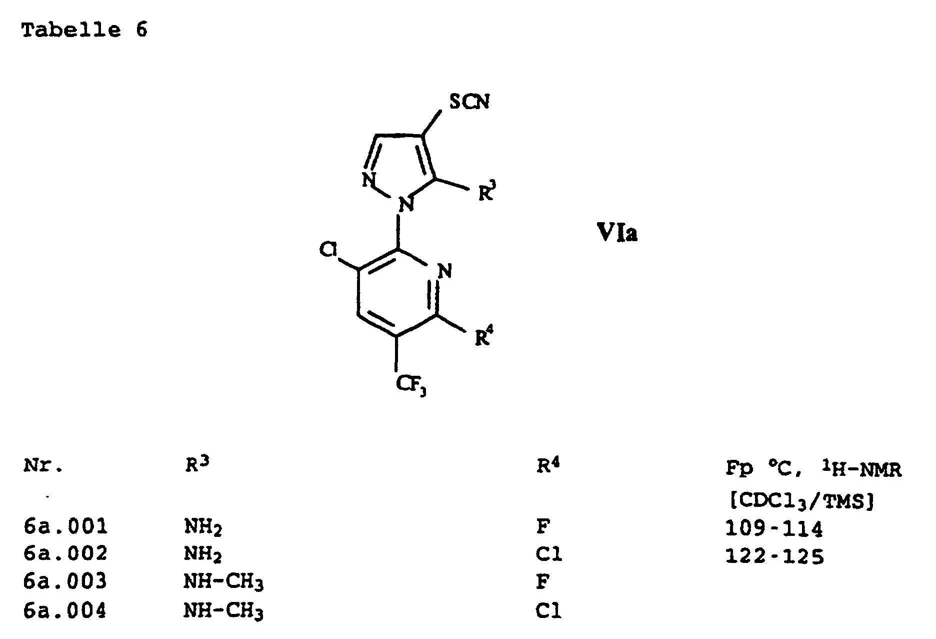

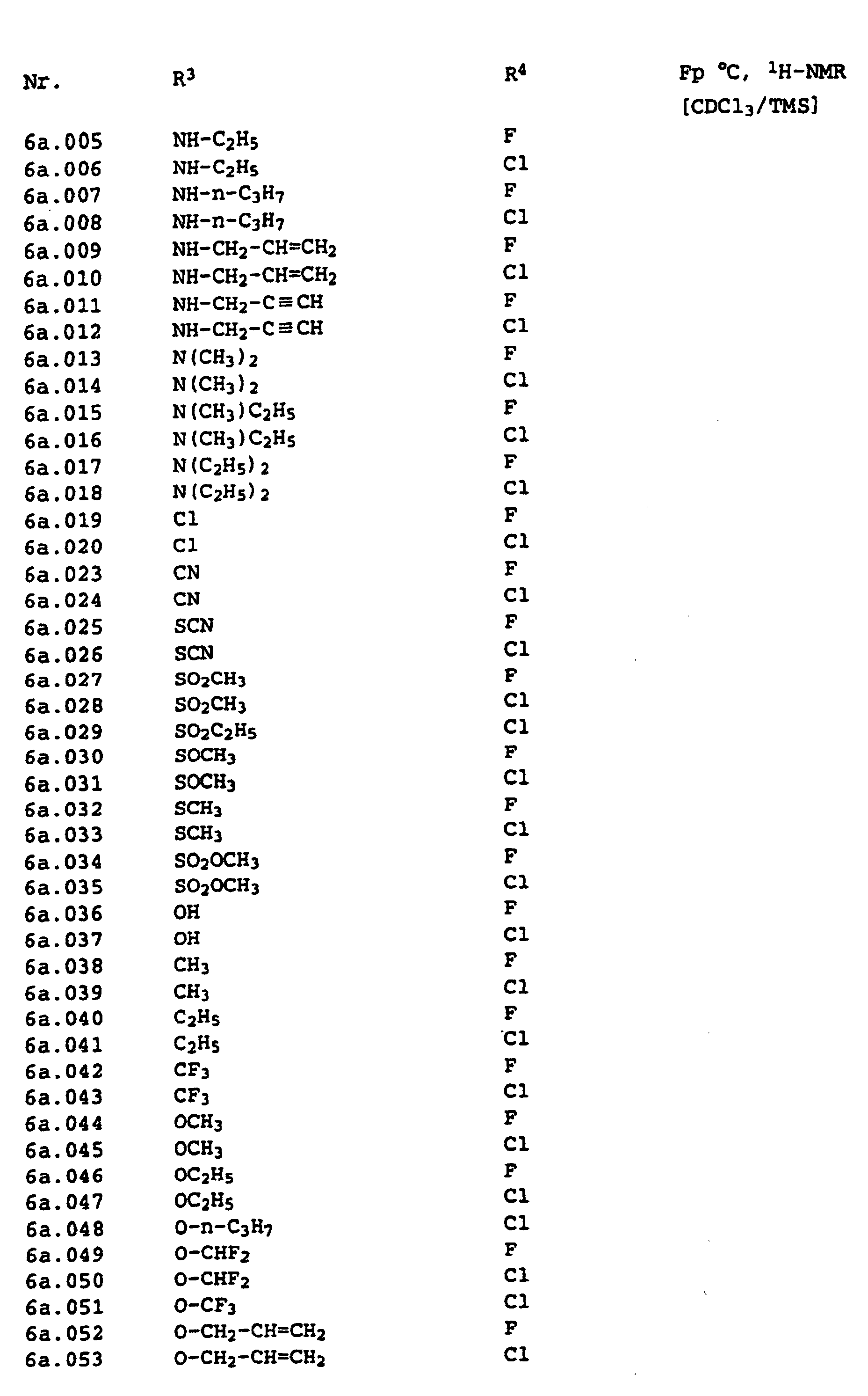

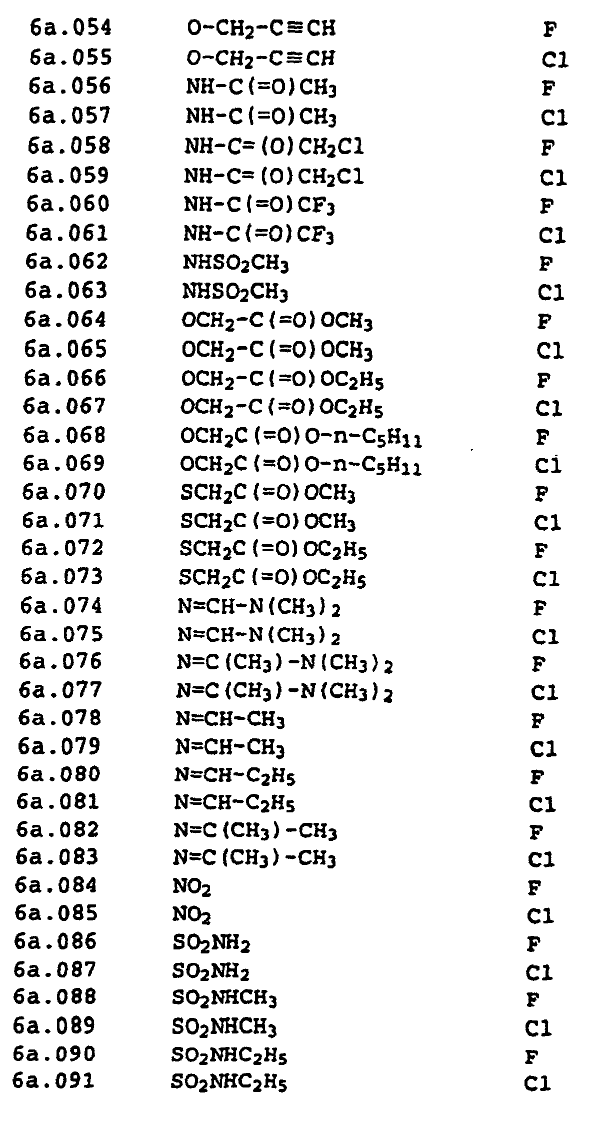

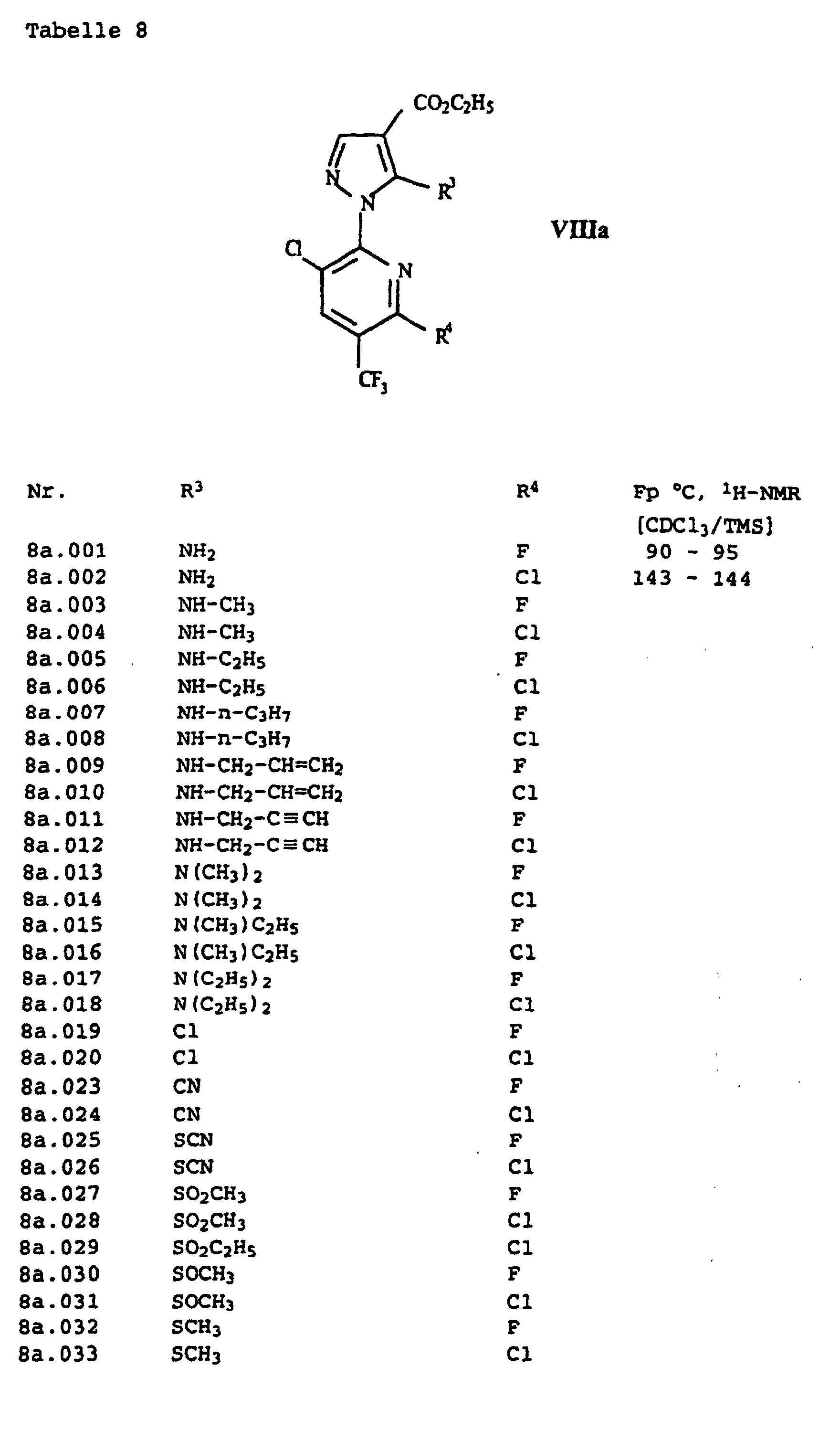

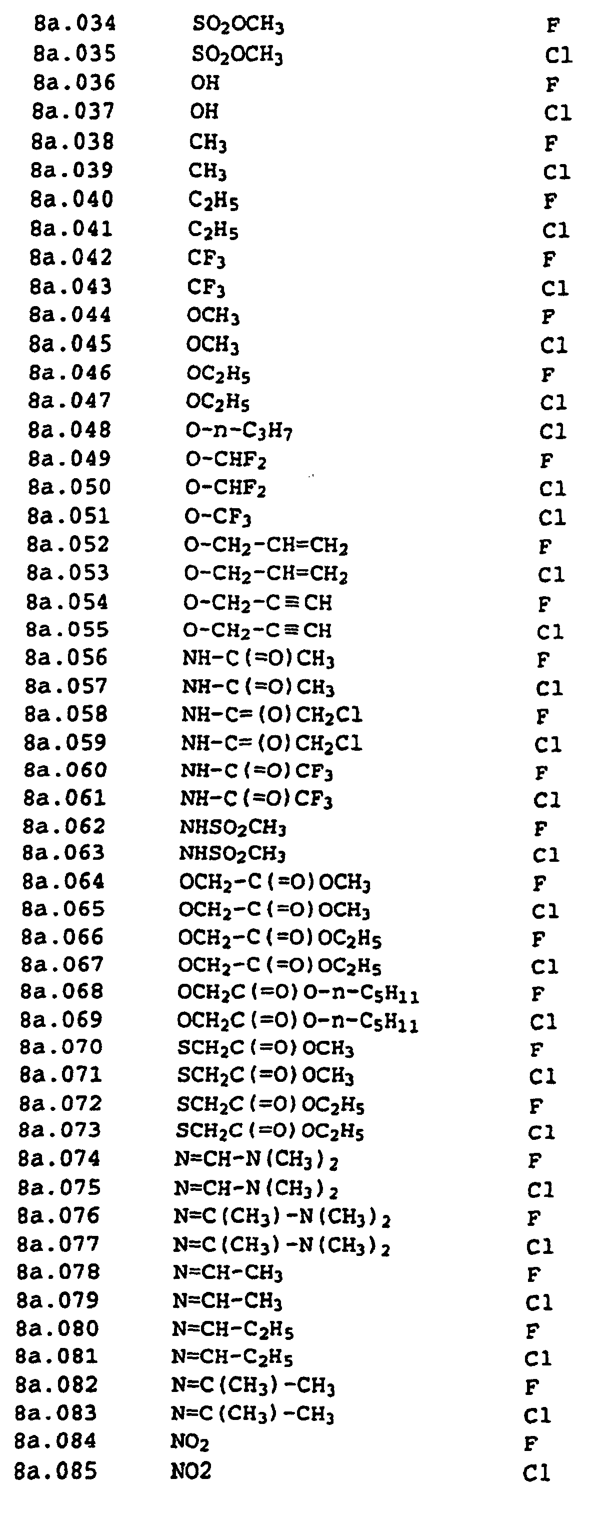

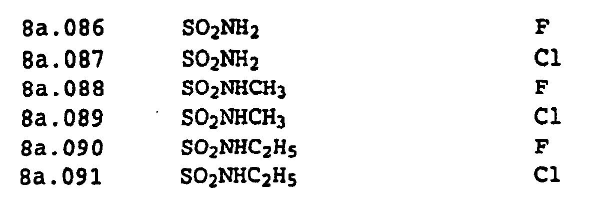

Des weiteren sind die folgenden substituierten 1-(Pyridyl)-pyrazole besonders bevorzugt:
- die Verbindungen der Formel Ib, Nr. 1b.001 - 1b.091, die sich von den entsprechenden Verbindungen der Formel Ia, Nr. 1a.001 - 1a.091 lediglich dadurch unterscheiden, daß die Substituenten Chlor und Trifluormethyl vertauscht sind: 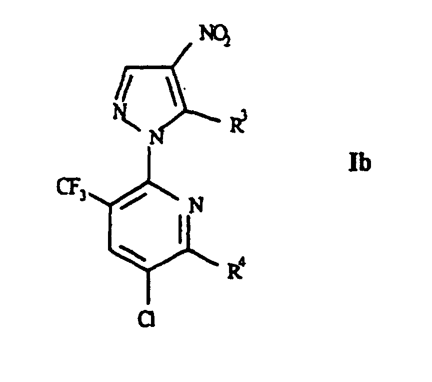
- die Verbindungen der Formel IIIb, 3b.001 - 3b.091, die sich von den entsprechenden Verbindungen der Formel IIIa, Nr. 3a.001 - 3a.091 lediglich dadurch unterscheiden, daß die Substituenten Chlor und Trifluormethyl vertauscht sind: 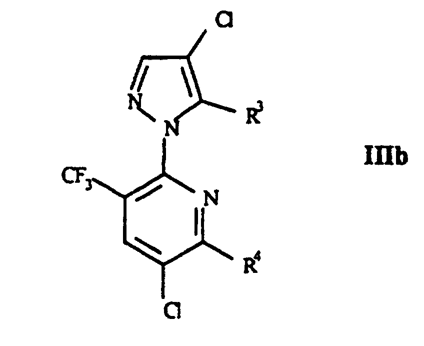
- die Verbindungen der Formel IVb, 4b.001 - 4b.091, die sich von den entsprechenden Verbindungen der Formel IVa, Nr. 4a.001 - 4a.091 lediglich dadurch unterscheiden, daß die Substituenten Chlor und Trifluormethyl vertauscht sind: 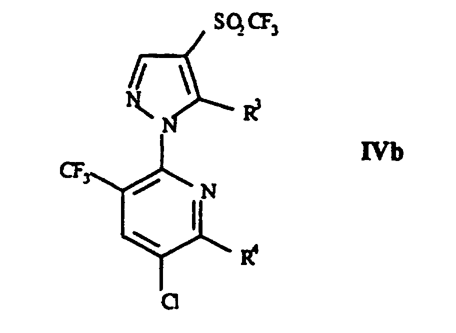
- die Verbindungen der Formel Vb, 5b.001 - Sb.091, die sich von den entsprechenden Verbindungen der Formel Va, Nr. 5a.001-Sa.091 lediglich dadurch unterscheiden, daß die Substituenten Chlor und Trifluormethyl vertauscht sind: 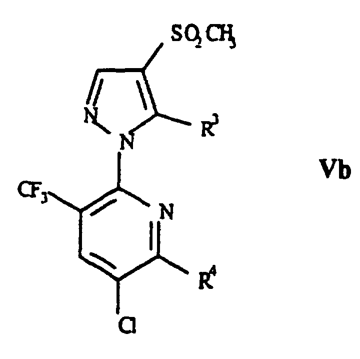
- die Verbindungen der Formel VIb, 6b.001 - 6b.091, die sich von den entsprechenden Verbindungen der Formel VIa, Nr. 6a.001- 6a.091 lediglich dadurch unterscheiden, daß die Substituenten Chlor und Trifluormethyl vertauscht sind: 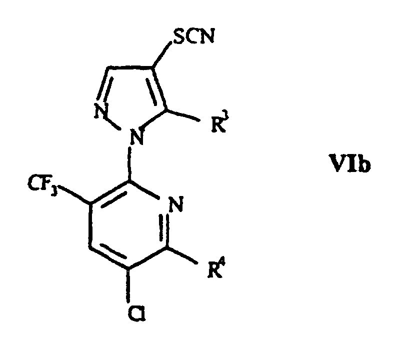
- die Verbindungen der Formel VIIIb, 8b.001 - 8b.091, die sich von den entsprechenden Verbindungen der Formel VIIIa, Nr. 8a.001 - 8a.091 lediglich dadurch unterscheiden, daß die Sub stituenten Chlor und Trifluormethyl vertauscht sind: 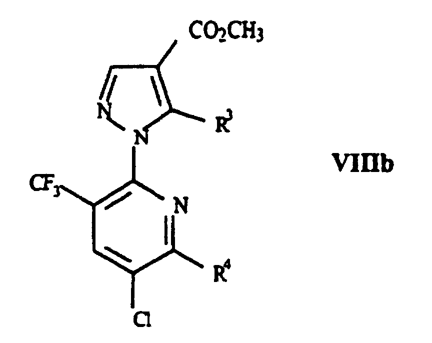
- die Verbindungen der Formeln Ic bzw. Id, die sich von den entsprechenden Verbindungen der Formeln Ia, Nr. 1a.001-1a.091 bzw. Ib, Nr. 1b.001 - 1b.091 lediglich dadurch unterscheiden, daß R⁴ Methoxy bedeutet: 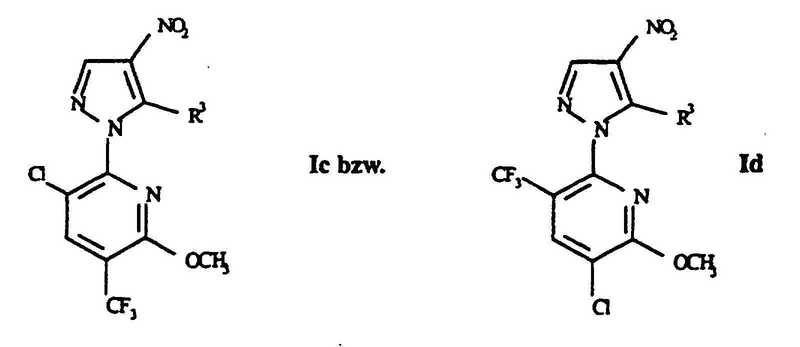
- die Verbindungen der Formeln IIIc bzw. IIId, die sich von den entsprechenden Verbindungen der Formeln IIIa, Nr. 3a.001-3a.091 bzw. IIIb, Nr. 3b.001 - 3b.091 lediglich dadurch unterscheiden, daß R⁴ Methoxy bedeutet: 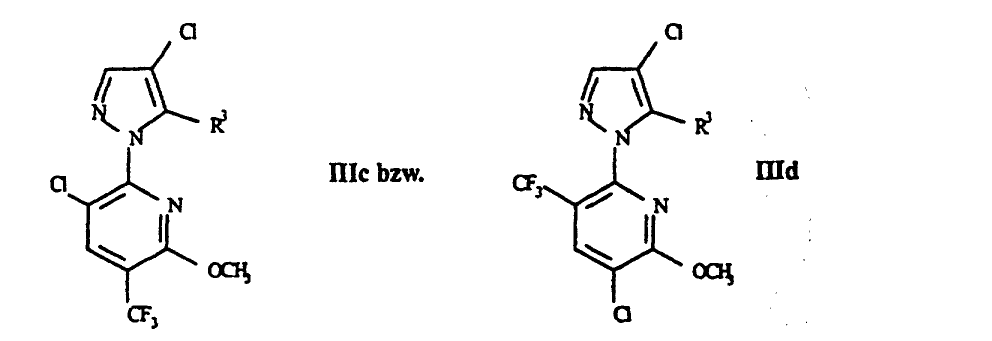
- die Verbindungen der Formeln IVc bzw. IVd, die sich von den entsprechenden Verbindungen der Formeln IVa, Nr. 4a.001-4a.091 bzw. IVb, Nr. 4b.001 - 4b.091 lediglich dadurch unterscheiden, daß R⁴ Methoxy bedeutet: 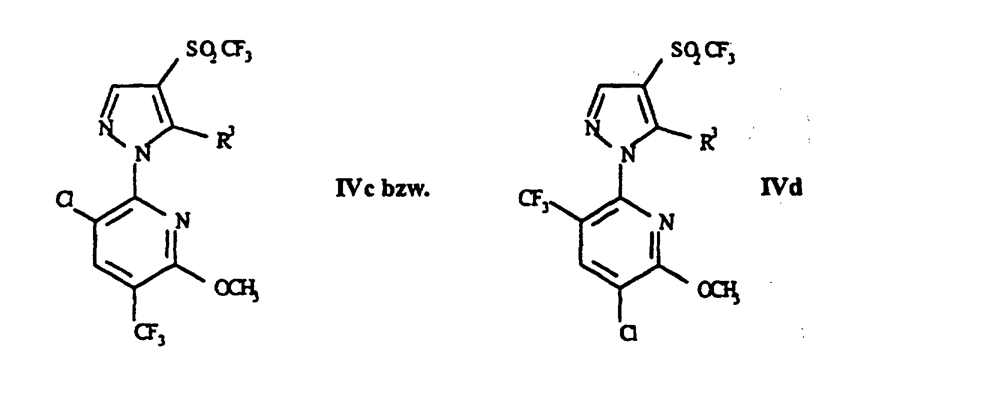
- die Verbindungen der Formeln Vc bzw. Vd, die sich von den entsprechenden Verbindungen der Formeln Va, Nr. 5a.001-5a.091 bzw. Vb, Nr. 5b.001 - 5b.091 lediglich dadurch unterscheiden, daß R⁴ Methoxy bedeutet: 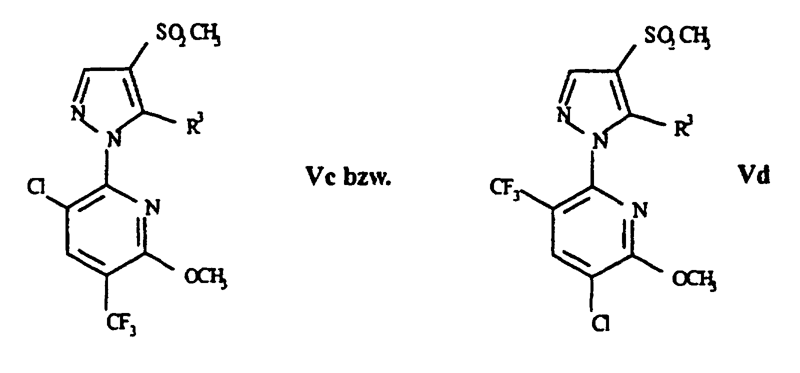
- die Verbindungen der Formeln VIc bzw. VId, die sich von den entsprechenden Verbindungen der Formeln VIa, Nr. 6a.001-6a.091 bzw. VIb, Nr. 6b.001 - 6b.091 lediglich dadurch unterscheiden, daß R⁴ Methoxy bedeutet: 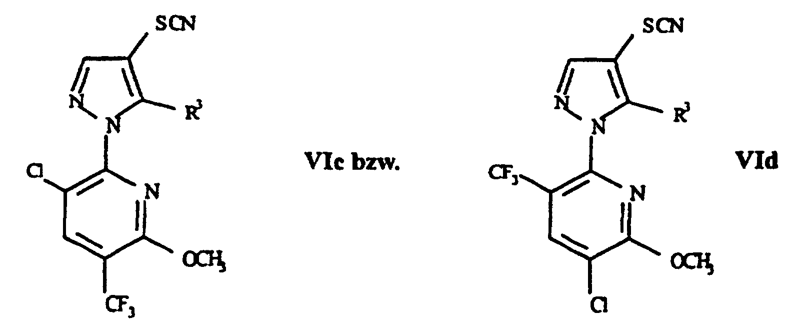
- die Verbindungen der Formeln VIIIc bzw. VIIId, die sich von den entsprechenden Verbindungen der Formeln VIIIa, Nr. 8a.001 - 8a.091 bzw. VIIIb, Nr. 8b.001 - 8b.091 lediglich dadurch unterscheiden, daß R⁴ Methoxy bedeutet: 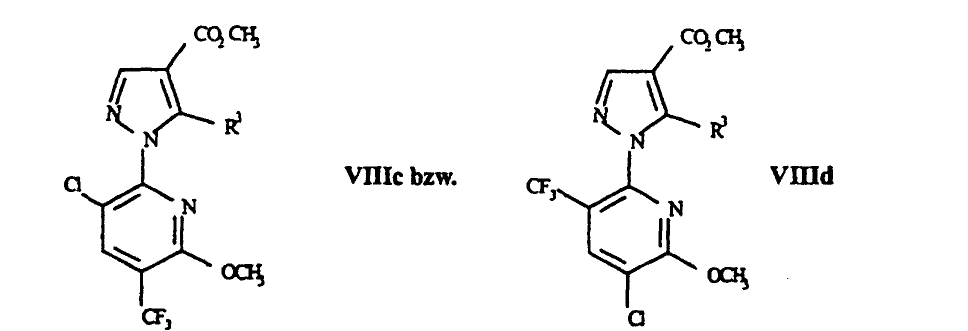
- sowie die in Tabelle 9 beschriebenen weiteren Verbindungen der Formel I

Die 1-(Pyridyl)-pyrazole der Formel I sind auf verschiedene Weise erhältlich, beispielsweise nach einem der folgenden Verfahren:

### Verfahren A:

Umsetzung eines Pyridyl-2-hydrazins der Formel IX, in der R⁴ bis R⁶ die vorgenannte Bedeutung haben, mit einem Acrylnitrilderivat der Formel X gemäß DBP 3520 330:

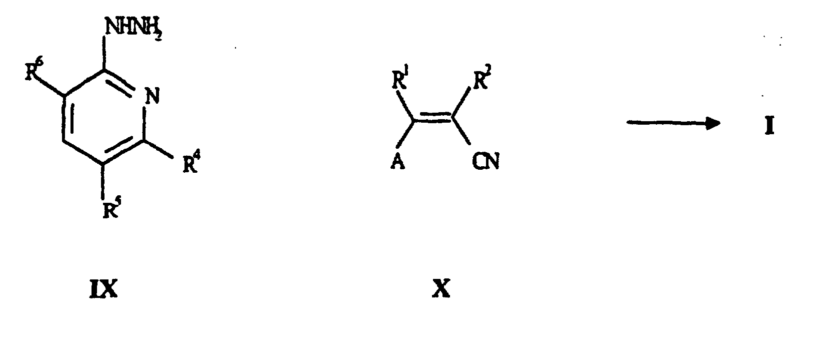

Hierbei haben R¹ und R² die oben angegebene Bedeutung und A steht für Halogen, Hydroxy, Alkoxy oder Dialkylamino.

Üblicherweise arbeitet man in einem inerten Lösungs- oder Verdünnungsmittel, insbesondere in einem halogenierten Kohlenwasserstoff wie Dichlormethan, 1,2-Dichlormethan, einem Alkohol wie Methanol, Ethanol, n-Propanol, Isopropanol, n-Butanol, Glykol, Methoxyethanol, Ethoxyethanol, Methoxypropanol oder einem Ether wie Tetrahydrofuran, Methyl-tert.-butylether, 1,2-Dimethoxyethan oder 1,2-Diethoxyethan. Die Reaktionstemperatur liegt normalerweise bei 0 - 180°C, bevorzugt 60 - 140°C.

Üblicherweise werden die Komponenten in etwa stöchiometrischen Mengen eingesetzt, jedoch kann ein Überschuß einer der Komponenten, z. B. im Hinblick auf eine möglichst vollständige Umsetzung der anderen Komponente, vorteilhaft sein.

Die Hydrazine der Trifluormethylpyridine der allgemeinen Formel IXa oder IXb 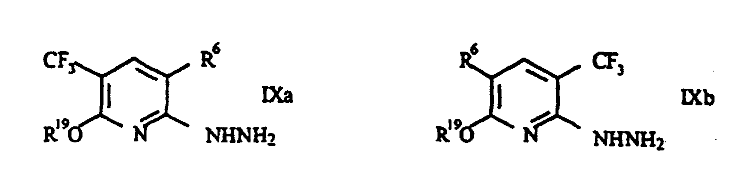 in der R⁶ und R¹⁹ die vorgenannte Bedeutung haben
erhält man beispielsweise, wenn man 2,3,6-Trichlor-5-trifluormethylpyridin der allgemeinen Formel XI 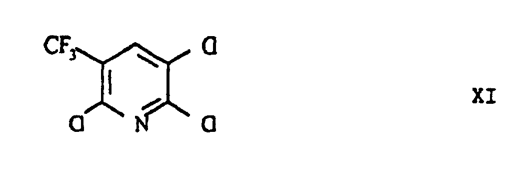 in einem ersten Schritt einem Halogenaustausch zu dem entsprechenden 3-Chlor-2,6-difluor-5-trifluormethylpyridin der folgenden Formel unterzieht XII 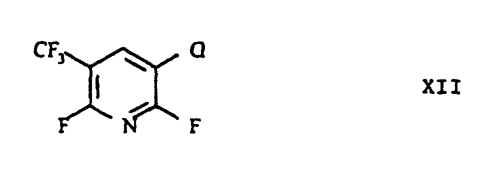 und dieses dann nacheinander mit Hydrazin zu dem 2-Hydrazino-3-chlor-6-fluor-5-trifluormethylpyridin der folgenden Formel XIII 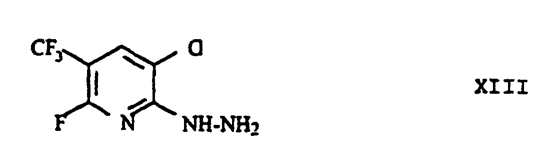 und schließlich mit einem Alkohol der allgemeinen Formel XIV

HO-R¹⁹ XIV

in der R¹⁹ die vorgenannte Bedeutung hat, oder dessen Alkali- oder Erdalkalisalz zu den Verbindungen der Formel IXa, in der R⁶ für Chlor steht und R¹⁹ die vorgenannte Bedeutung hat, umsetzt, oder wenn man zur Darstellung der Verbindungen der Formel IXb, in der R⁶ für Chlor steht und R¹⁹ die vorgenannte Bedeutung hat, das oben erhaltene 3-Chlor-2,6-difluor-5-trifluormethylpyridin der folgenden Formel XII 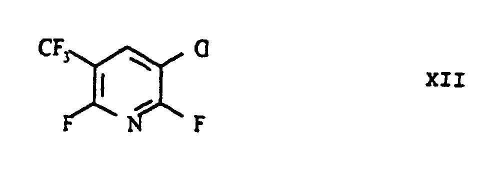 nacheinander zuerst mit einem Alkohol der folgenden Formel XIV,

HO-R¹⁹ XIV

in der R¹⁹ die vorgenannte Bedeutung hat, oder dessen Alkali- oder Erdalkalisalz zu den Verbindungen der folgenden Formel 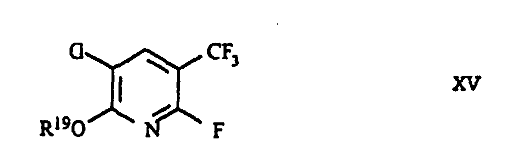 und zum Schluß mit Hydrazin umsetzt.

Weiterhin wurde gefunden, daß man die Verbindungen der Formel IXb auch dadurch erhalten kann, daß man zuerst das 2,3,6-Trichlor-5-trifluormethylpyridin der folgenden Formel XI 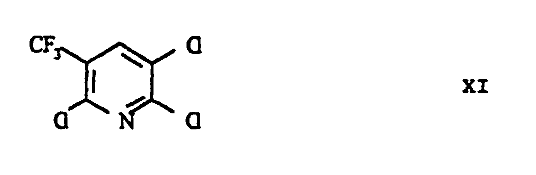 mit einem Alkohol der folgenden Formel XIV

HO-R¹⁹ XIV

in der R¹⁹ die vorgenannte Bedeutung hat, zu den Verbindungen der allgemeinen Formel XVI 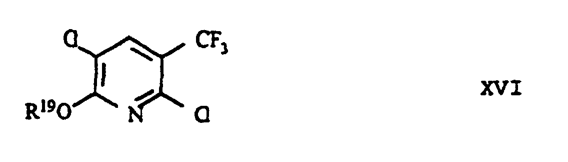 umsetzt, diese dann einem Halogenaustausch zu den Verbindungen der folgenden Formel XV 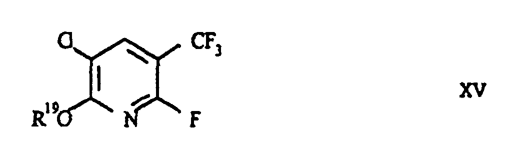 in der R¹⁹ die vorgenannte Bedeutung hat, unterzieht und diese zuletzt wie beschrieben mit Hydrazin zu den Endstoffen IXb, bei denen R⁶ für Chlor steht und R¹⁹ die vorgenannte Bedeutung hat, umsetzt.

Weiterhin wurde im Falle der Darstellung der 2-Alkoxy-3-chlor-6-hydrazino-5-trifluormethyl-pyridine sowie ihrer ungesättigten Analogen als weiterer Vertreter der Zwischenprodukte IXb gefunden, daß man diese auch dadurch erhalten kann, daß man die Verbindungen der allgemeinen Formel XVI 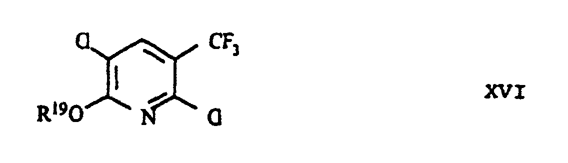 in der R¹⁹ die vorgenannte Bedeutung hat, direkt mit Hydrazin umsetzt, ohne vorher einen Halogenaustausch durchzuführen.

Die Umsetzung kann im Falle der Darstellung der Verbindungen IXa unter Verwendung von 2,3,6-Trichlor-5-trifluormethylpyridin und Kaliumfluorid als Halogenaustauschmittel sowie der Nucleophile Hydrazin und Methanol durch folgendes Schema beschrieben werden:

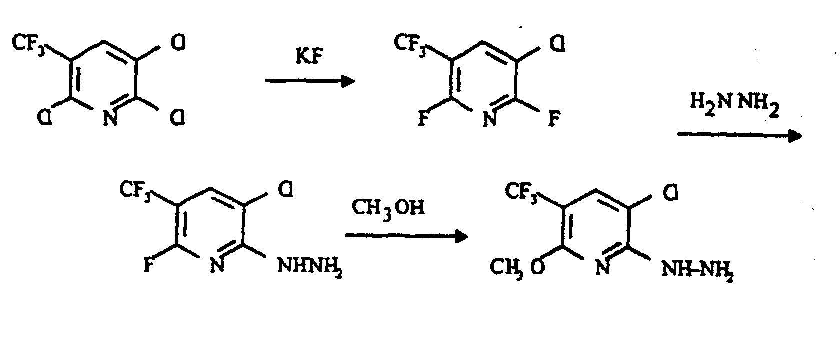

Im Falle der Darstellung der Verbindungen IXb verläuft die Umsetzung ebenfalls auf Basis von 3-Chlor-2,6-difluor-5-trifluormethylpyridin unter Verwendung von beispielsweise Methanol und Hydrazin nach folgendem Schema:

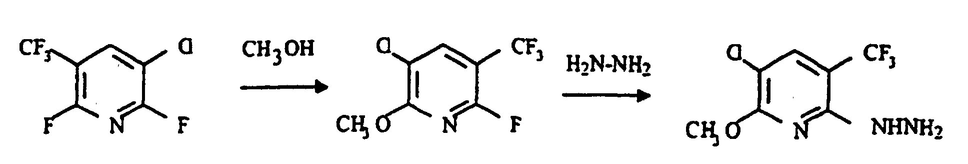

Alternativ kann man zur Darstellung der Verbindungen IXb auch von dem 2,3,6-Trichlor-5-trifluormethylpyridin und beispielsweise Propagylalkohol ausgehen, dann einen Halogenaustausch ausführen und zum Schluß mit Hydrazin nach folgendem Schema umsetzen:

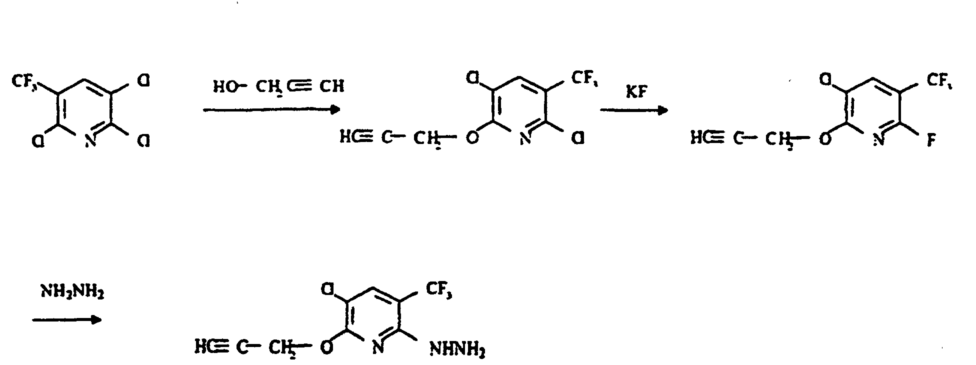

Das Verfahren liefert auf einfachem und wirtschaftlichem Weg neue 2-Hydrazino-halogen-trifluormethylpyridine in hoher Ausbeute und Reinheit. Entgegen der Erwartung wird die Trifluormethylgruppe nicht verseift noch eine bereits eingeführte Alkoxygruppe durch Ammoniak wieder eliminiert.

Die neuen substituierten Pyridine IXa und IXb sind auf verschiedene Weise erhältlich, vorzugsweise nach einem der folgenden Verfahren:

Der Halogenaustausch des 2,3,6-Trichlor-5-trifluormethylpyridins wird bevorzugt in Gegenwart eines polaren Lösungsmittels mit Kaliumfluorid bei Temperaturen zwischen 100 - 180°C vorzugsweise 130 - 170°C durchgeführt.

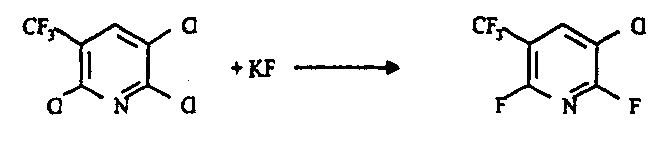

Als Lösungsmittel verwendet man für diese Umsetzungen Nitrile wie Acetonitril, Propionitril, Butyronitril, Isobutyronitril, Glykolether wie Dimethylglykolether. Diethylglykolether, Diethylenglykoldimethylether, carbonsäureamide wie DMF, N-Methylpyrrolidon, Harnstoffe wie Tetraethylharnstoff, Tetrabutylharnstoff, Dimethylethylenharnstoff, Dimethylpropylenharnstoff, Sulfoxide wie Dimethylsulfoxid und vorzugsweise Sulfone wie Dimethylsulfon, Diethylsulfon, Tetramethylensulfon (Sulfolan) oder Pentamethylensulfon. Auch die Durchführung in der Schmelze ohne Zusatz eines Lösungsmittels ist erfindungsgemäß möglich.

Der Halogenaustausch verläuft bereits ohne Katalysator mit hoher Geschwindigkeit. Er kann jedoch durch Zugabe eines Katalysators, z. B. eines Kronenethers oder Cryptanden beschleunigt werden. Diese sind organische Komplexliganden, die insbesondere gut zur Bindung von Alkali dienen. Die Cryptanden liefern eine dreidimensionale Umhüllung. Bezüglich Herstellung dieser Stoffe, siehe "Kontakte" (1977), Seiten 11 - 31 und 36 - 48. Als Katalysator sind Kronenether bevorzugt, von denen z. B. die folgenden Verbindungen genannt sein sollen: 12-Krone-4, 14-Krone-4, Dibenzo-14-krone-4, 18-Krone-5, 18-Krone-6, Dibenzo-18-krone-6 oder Aza-18-krone-6.

Diese Katalysatoren werden zweckmäßig in einer Menge von 0,05-5, insbesondere 0,1 - 2 Molprozent je Mol Ausgangsstoff eingesetzt.

Die molaren Verhältnisse, in denen die Ausgangsverbindungen miteinander umgesetzt werden, betragen 1,9 - 2,8, vorzugsweise 2 - 2,4 für das Verhältnis von Kaliumfluorid zu Pyridinderivat. Die Konzentration der Edukte im Lösungsmittel beträgt 0,1 - 5 mol/l, bevorzugt 0,2 - 2 mol/l.

Besonders vorteilhaft läßt sich die Verbindung 3-Chlor-2,6-difluor-5-trifluormethylpyridin herstellen, wenn man vor dem eigentlichen Halogenaustausch das 2,3,6-Trichlor-5-trifluormethylpyridin beispielsweise in Gegenwart eines aliphatischen Sulfons bei Temperaturen bis 150°C. zweckmäßig zwischen 50°C und 120°C, insbesondere 70 - 100°C mit 0,1 - 0,4, zweckmäßig 0,15 - 0,3 mol eines Säurechlorids der schwefligen Säure oder Kohlensäure behandelt und das Reaktionsgemisch dann bei Temperaturen von 70-250°C, vorzugsweise 80 - 200°C mit Kaliumfluorid umsetzt.

Als Katalysatoren sind für diese Verfahrensstufe z. B. N,N-disubstituierte Carbonamide wie DMF, N,N-Dimethylacetamid oder N,N-Diisopropylacetamid geeignet. Der Katalysator wird zweckmäßig in einer Menge von 0,2 - 2 Gewichtsprozent bezogen auf das Säurechlorid eingesetzt.

Bei der Umsetzung mit dem Säurechlorid erhitzt man zweckmäßig so lange, bis keine Gasentwicklung mehr erfolgt. Es empfiehlt sich, überschüssiges Säurechlorid z. B. durch Einblasen eines Inertgases, wie Stickstoff, oder durch Anlegen von Vakuum zu entfernen.

Zu diesem Gemisch gibt man dann das Kaliumfluorid, das zweckmäßig vorher getrocknet wurde und hält die durch Verrühren erhaltene Mischung 1 - 10 Stunden auf Reaktionstemperatur.

Als Fluoridsalze kommen erfindungsgemäß neben Kaliumfluorid auch Tetraalkyl-(C₁-C₁₃)-ammoniumfluorid sowie entsprechende Mischungen untereinander oder mit Cäsiumfluorid oder Rubidiumfluorid in Frage, wobei diese Mischungen mit Cäsiumfluorid nicht mehr als 50 Gew.-% an Cäsiumfluorid enthalten. Bevorzugt werden Fluoridmischungen verwendet, die zumindest 75 Gew.-% Kaliumfluorid enthalten; insbesondere bestehen derartige Mischungen aus wenigstens 90 Gew.-% Kaliumfluorid und höchstens 10 Gew.-% Cäsiumfluorid oder aus 60 Gew.-% Kaliumfluorid und 40 Gew.-% Rubidiumfluorid. In einer weiteren bevorzugten Ausgestaltungsform kommt als Fluoridsalz nur Kaliumfluorid zum Einsatz.

Als Phasentransferkatalysatoren können quartäre Ammonium- oder Phosphoniumsalze verwendet werden. An geeigneten Verbindungen seien folgende genannte: Tetraalkyl-(C₁-C₁₈)-ammoniumchloride, -bromide oder -fluoride, Tetraalkyl-(C₁-C₁₈)-phosphoniumchloride oder -bromide, Tetraphenylphosphoniumchlorid oder -bromid, (Phenyl)ₘ (alkyl-(C₁-C₁₈))ₙ-phosphoniumchloride oder -bromide, wobei m = 1 - 3, n = 3 - 1 und m + n = 4 ist. Auch Gemische dieser Salze sind einsetzbar. Bei geeigneter Wahl des Katalysators für die jeweilige umzusetzende Verbindung, was durch einige Routineversuche leicht zu ermitteln ist, erhält man hohe Raumleistungen und Ausbeuten; außerdem sind die Apparatetotzeiten sowie die gesamten Apparatekosten nach dem erfindungsgemäßen Verfahren besonders günstig.

Die Menge an Phasentransferkatalysator beträgt im allgemeinen bis zu 20 Gew.-%, bevorzugt zwischen 3 und 15 Gew.-% und besonders bevorzugt zwischen 3 - 8 Gew.-%, bezogen auf die eingesetzte Menge an Fluoridsalz.

Als Phasentransferkatalysatoren können auch Oligo- oder Polyalkylenglykoldimethylether verwendet werden, wobei der Alkylenrest 2 - 6 C-Atome, vorzugsweise 2 und / oder 3-C-Atome enthält, also vorzugsweise den Ethylen- und / oder den Propylenrest und insbesondere nur den Ethylenrest darstellt. Die Zahl der O-Ethylen(glykol)einheiten (-O-CH₂-CH₂-)ₙ und / oder der O-Propyleneinheiten in diesen Verbindungen kann von n = 4 (z. B. Tetraethylenglykoldimethylether) bis etwa n = 150 betragen; bevorzugt werden jedoch Ether eingesetzt, deren Polymerisationsgrad zwischen n = 4 und n = 25 beträgt. Im Falle von Alkylenresten mit mehr als 3 C-Atomen liegt n im allgemeinen nicht höher als 6. Die Einsatzmenge dieser Ether, insbesondere Glykolether liegt zumeist zwischen etwa 0,6 Gew.-% und etwa 200 Gew.-%, bevorzugt zwischen etwa 5 und etwa 100 Gew.-% und besonders bevorzugt zwischen etwa 10 und etwa 50 Gew.-%, bezogen auf die Menge des eingesetzten Fluoridsalzes. Der besondere Vorteil bei der Verwendung dieser Verbindungen liegt darin, daß meist der Einsatzmenge entsprechend weniger Lösungsmittel verwendet werden kann, da die Glykolether bei der Reaktionstemperatur im allgemeinen flüssig werden. Auch Gemische dieser Ether untereinander als auch Gemische dieser Ether (einzeln oder im Gemische) mit den quartären Ammonium- oder Phosphoniumsalzen, vorzugsweise Glykolether mit quartären Phosphoniumsalzen, können eingesetzt werden.

Verwendet man als Fluoridsalz Tetraalkyl-(C₁-C₁₈)-ammoniumfluoride, so ist der Zusatz eines weiteren Phasentransferkatalysators nicht erforderlich, da das Fluoridsalz selbst einen solchen darstellt, der damit also in stöchiometrischen und größeren Mengen eingesetzt werden kann.

Die Verwendung von sprühgetrocknetem Alkalimetallfluorid im erfindungsgemäßen Verfahren verkürzt dabei zwar zum Teil die Reaktionszeiten, ist aber nicht unbedingt notwendig. Ebenso kann unter Zusatz von säurebindenden Mitteln, wie Alkali- und Erdalkalimetallcarbonaten oder basisch wirkenden Oxiden, wie zum Beispiel Magnesiumoxid, oder entsprechenden Gemischen, gearbeitet werden. Besonders bevorzugt ist hierbei Kaliumcarbonat, das in Anteilen von etwa 1 - etwa 10 Gew.-%, bevorzugt von etwa 4 - etwa 6 Gew.-%, bezogen auf die Fluoridsalz-Menge, verwendet wird.

Die säurebindende Mittel sind für den Reaktionsverlauf im allgemeinen nicht wesentlich. In einigen Fällen wird die Reaktionsgeschwindigkeit durch die Bildung von Fluorwasserstoff während der Reaktion erheblich verringert. In diesen Fällen ist es günstig, besonders auch zur Vermeidung von Apparatekorrosion, unter Anwesenheit von derartigen Säurefängern zu arbeiten. Der Einsatz dieser Verbindungen bei Fraktionierung des Reaktionsgemisches oder des Rohproduktes kann aus Gründen der Korrosion in der Fraktionieranlage wünschenswert sein, wobei Magnesiumoxid hierbei besonders bevorzugt ist. Zu diesem Zweck werden der Fraktionierblase bis etwa 10 Gew.-% an Säurefänger zugesetzt, bevorzugt zwischen etwa 3 und 8 Gew.-%, bezogen auf die Gesamtmenge an eingesetzten Destillationssumpf.

Nach der Umsetzung mit Alkalifluorid arbeitet man auf an sich übliche Weise, z. B. durch Filtration, Waschen des Festgutes, Destillation von Filtrat und Waschfiltraten, auf. Im Falle von wassermischbaren Lösungsmitteln kann man die Pyridinderivate XII oder XV auch durch Zugabe von Wasser ausfällen und wie beschrieben aufarbeiten.

Die Umsetzung des 2,6-Difluorpyridins und der 2-Fluorpyridine mit Hydrazin bzw. der 2,5-Dichlorpyridine mit Hydrazin kann in Abwesenheit oder vorteilhaft in Gegenwart eines Lösungsmittels vorgenommen werden. Als Lösungsmittel sind insbesondere die nachfolgend aufgeführten geeignet:

Kohlenwasserstoffe, z. B. Pentan, Hexan, Heptan, Cyclohexan, Alkohole, z. B. Methanol, Ethanol, n-Propanol, Isopropanol, Butanol, Isobutanol, Ether wie Methyl-tert.-butylether, Diethylether, Ethylpropylether, n-Butylethylether, Di-n-Butylether, Diisobutylether, Diisoamylether, Di-isopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat, chlorierte Kohenwasserstoffe wie Methylenchlorid, 1,1,2,2,-Tetrachlorethan, 1,1-Dichlorethylen, 1,2-Dichlorethan, Chlorbenzol, 1,2-, 1,3-, 1,4-Dichlorbenzol, 1-Chlornaphthalin und 1,2,4-Trichlorbenzol, Nitrokohlenwasserstoffe wie Nitromethan, Nitroethan, Nitropropan und Nitrobenzol, dipolare aprotische Lösungsmittel, z. B. Acetonitril, Propionitril, Dimethylformamid, Dimethylacetamid, Dimethylsulfoxid, N-Methylpyrrolidon, 1,3-Dimethyltetrahydro-2-(1H)-pyrimidinon und 1,3-Dimethyl-imidazolidin-2-on, Aromaten, z. B. Benzol, Toluol und Xylol oder Heteroaromaten, z. B. Pyridin, α,β,γ-Picolin und Chinolin, oder Wasser und Gemische dieser Lösungsmittel.

Zweckmäßig verwendet man das Lösungsmittel in einer Menge von 100 - 2000 Gew.-%, vorzugsweise 400 - 1200 Gew.-%, bezogen auf die Ausgangsstoffe XII, XV oder XVI.

Vorteilhaft gibt man 0,9 - 10, insbesondere 1.1 - 5 Molequivalent Hydrazinhydrat, bezogen auf die Ausgangsstoffe, innerhalb 0,25-2 Stunden zu einer Mischung der Ausgangstoffe in einem der vorgenannten Lösungsmittel bei 0 - 180°C, vorzugsweise 10 - 130°C und rührt bis zur Vervollständigung der Reaktion (ca. 2 - 20 Stunden) nach.

Setzt man nur ungefähr stöchiometrische Mengen Hydrazin ein, so verwendet man zweckmäßig zusätzlich eine organische Hilfsbase, um den entstehenden Halogenwasserstoff abzufangen. Als Hilfbase eignen sich dazu übliche organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α,β,γ-Picolin, 2,4- und 2,6-Lutidin und Triethylendiamin. Als Hilfsbase kann man jedoch auch anorganische basische Stoffe z. B. ein Alkali- oder Erdalkalihydroxid wie Lithium-, Natrium-, Kalium-, Calcium-, Magnesium-, oder Zinkhydroxid oder ein Alkali- oder Erdalkalihydrogencarbonat oder-Carbonat der gleichen oben genannten Kationen verwenden. Im allgemeinen sind Zusätze von 0,9 - 1,1 Equivalenten der Hilfsbase, bezogen auf die Ausgangsstoffe XII, XV oder XVI ausreichend.

Die Reaktion kann drucklos oder unter Druck, kontinuierlich oder diskontinuierlich durchgeführt werden.

Die Aufarbeitung kann in üblicher Weise erfolgen, z. B. extrahiert man das Umsetzungsgemisch mit Wasser zur Entfernung der Salze, trocknet und reinigt die organische Phase, z. B. durch Chromatographie oder Destillation. Man kann jedoch auch direkt die organische Phase einengen und den Rückstand mit einem Lösungsmittel verrühren.

Die Umsetzung der 2,6-Dichlorpyridine, der 2,6-Difluorpyridine und der 2-Hydrazino-pyridine mit einem Alkohol oder dessen Salz kann in Abwesenheit oder vorteilhaft in Gegenwart eines Lösungsmittels vorgenommen werden. Als Lösungsmittel können die vorgenannten eingesetzt werden, ferner Ketone, z. B. Aceton, Methylethylketon oder entsprechende Gemische; man kann jedoch auch den verwendeten Alkohol direkt als Lösungsmittel verwenden.

Zweckmäßig verwendet man das Lösungsmittel in einer Menge von 100 - 2000 Gew.-%, vorzugsweise 400 - 1200 Gew.-%, bezogen auf die Ausgangsstoffe XI, XII oder XIII.

Zur Bindung des bei der Reaktion abgespaltenen Halogenwasserstoffs setzt man zweckmäßig ein Alkali- oder Erdalkalihydroxid wie Lithium- Natrium-,Kalium-, Calcium-, Magnesium- oder Zinkhydroxid, ein Alkali oder Erdalkalihydrogencarbonat oder -carbonat der gleichen oben genannten Kationen oder ein Metallalkoholat, beispielsweise ein Lithium, Natrium oder Kaliumalkoholat zu. Die Alkoholate stellt man zweckmäßig in situ durch Auflösen obengenannter Metalle in dem einzusetzenden Alkohol oder durch Einwirkung von Lithium-, Natrium-, Kalium-, oder Calciumhydrid dar. Man kann jedoch auch eine der vorgenannten organischen Hilfsbasen verwenden.

Vorteilhaft gibt man 0,8 - 1,5, insbesondere 0,9 - 1,2 Molequivalent des Alkohols zweckmäßig in Gegenwart einer equivalenten Menge (einer Base), oder des entsprechenden Alkoholats innerhalb 0,25 - 0,5 Stunden zu einer Mischung der Ausgangstoffe XI, XII oder XIII in einem der vorgenannten Lösungsmittel bei -20 - 100°C, vorzugsweise 0 - 30°C und rührt bis zur Vervollständigung der Reaktion noch 1 - 12 Stunden bei 10 - 120°C vorzugsweise 2 - 10 Stunden bei 20 - 80°C nach und arbeitet dann auf. Man kann jedoch auch umgekehrt den Alkohol zusammen mit einer Base bzw. dem entsprechenden Alkoholat in dem Lösungsmittel vorgeben und dann die Ausgangsstoffe unter den obigen Bedingungen zugeben.

Die Reaktion kann drucklos oder unter Druck, kontinuierlich oder diskontinuierlich durchgeführt werden.

Die Aufarbeitung kann in gleicher Weise wie für die Umsetzung der Ausgangsstoffe beschrieben, durchgeführt werden.

### Verfahren B:

Hydrolyse und Decarboxylierung eines 1-(Pyridyl-2)-pyrazol-4-carbonesters der Formel Ie, in der R¹ bis R⁶ die vorgenannte Bedeutung haben, gemäß DBP 3520 330.

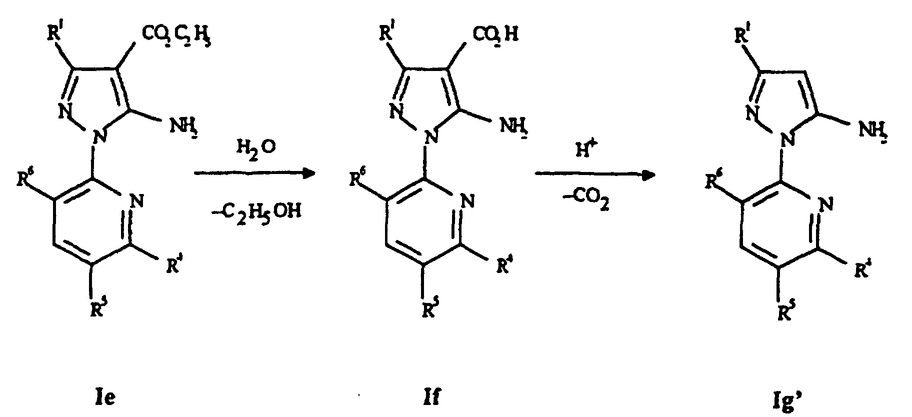

Hierzu kann man einen 1-(Pyridyl-2)-pyrazol-4-carbonester mit verdünnter wäßriger Alkalilauge ggf. in Gegenwart wäßrigen Alkohols als Lösungsvermittler bei 40 - 100°C, vorteilhaft 70 - 90°C während 1 - 6 Stunden hydrolisieren und dann durch Ansäuren die Carbsäure If isolieren. Durch Behandlung mit verdünnter Halogenwasserstoffsäure, zweckmäßig in Gegenwart eines niederen Alkohols als Lösungsvermittler bei 60 - 120°C, vorteilhaft 70 - 90°C erhält man dann das Pyrazolderivat Ig'.

Mann kann jedoch auch direkt den Pyrazolester der Formel Ie mit wäßriger Bromwasserstoffsäure bei 60 - 120°C, vorteilhaft 70-90°C behandeln, bis die verseifung und Decarboxylierung zu Ig' beendet ist.

Als Alkalilauge ist Natron- und Kalilauge geeignet.
Als Alkohole kommen Methanol, Ethanol, n-Propanol, Isopropanol oder n-Butanol in Betracht.

Als Halogenwasserstoffsäure sind Chlor-, Brom-, oder Jodwasserstoffsäure geeignet.

### Verfahren C:

Umsetzung eines 1-(Pyridyl-2)-5-aminopyrazols Ig mit einer reaktiven Schwefelhalogenverbindung XVII oder einer reaktiven Kohlensäureverbindung XVII oder einem reaktiven Amidacetal XIX 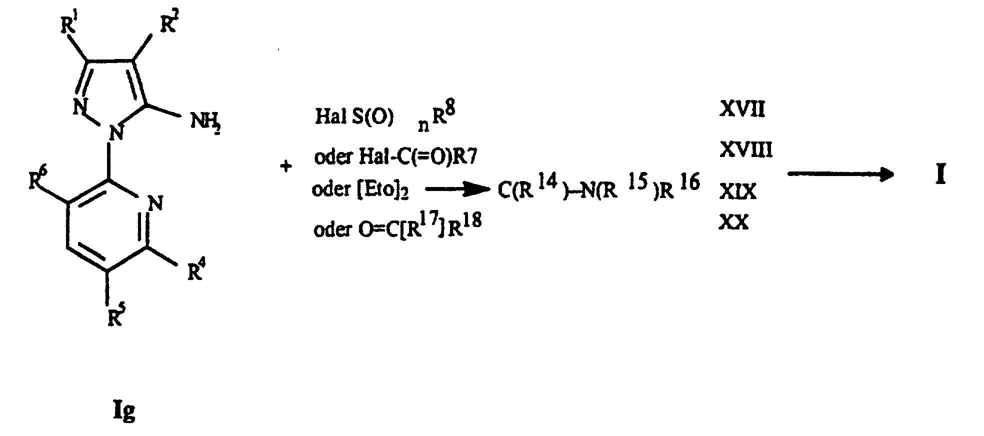 oder einer reaktiven Ketoverbindung XX gemäß DBP 3520330:

In den Formeln Ig, stellvertretend für I und XVII bis XX stehen R¹ bis R¹⁸ vorzugsweise für diejenigen Reste, die bereits in Zusammenhang mit der Beschreibung der erfindungsgemäßen Stoffe der Formel I als bevorzugt für diese Substitution genannt wurden. Halogen steht für Fluor, Chlor, Brom, vorzugsweise Chlor oder Brom.

Üblicherweise arbeitet man in einem inerten Lösungs- oder Verdünnungsmittel, insbesondere einem halogenierten Kohlenwasserstoff wie Dichlormethan, 1,2-Dichlorethan, einem Ether wie Diethylether, Methyl-tert.-butylether, Tetrahydrofuran, 1,4-Dioxan oder Anisol, einem Nitril wie Acetonitril, Propionitril oder Butyronitril, einem Ester wie Essigester, Propionsäuremethyl- oder Ethylester oder, falls die Anwesenheit von Wasser nicht stört, ggf. auch in einem Zweiphasengemisch mit Wasser.

Zur Bindung des, bei der Reaktion mit den Ausgangsstoffen XVII-XVIII freiwerdenden Halogenwasserstoffs setzt man zweckmäßig Basen wie beispielsweise Alkalimetallcarbonate und -hydrogencarbonate wie Natriumhydrogencarbonat, Kaliumhydrogencarbonat. Natriumcarbonat und Kaliumcarbonat, Alkalimetallalkoholate wie Natriummethanolat und Kalium-tert.-butanolat, Alkalimetallhydroxide wie Natriumhydroxid oder Kaliumhydroxid ein. Vorteilhaft kommen auch organische Basen in Frage: Trimethylamin, Triethylamin, Pyridin, α, β, γ-Picolin, Lutidin, N-Dimethylanilin, N-Diäthylanilin, N-Propyl-piperidin, Chinolin, Isochinolin, Chinazolin, Chinoxalin, Triäthanolamin, Triamylamin, Tri-n-butylamin, Trifurfurylamin, Trihexylamin, N-Methylimidazol, N-Methylpyrrol, N-Äthylpiperidin, N-Methylpyrrolidin, Pyrazin, Pyrimidin, Acridin, Phenanthridin, Phenazin, N-Dimethylcyclohexylamin oder N-Propyl-diisopropylamin.

Bei der Umsetzung der reaktiven Amidacetale XIX oder der Ketoverbindung XX genügt das Rühren der Ausgangsstoffe, ggf. unter Entfernung des abgespaltenen Alkohols bzw. des Reaktionswassers, durch Destillation bzw. Erwärmen an einem Wasserabscheider. Die Reaktionstemperatur liegt normalerweise bei 0 - 120°C, vorzugsweise bei 20 - 80°C.

Üblicherweise werden die Komponenten in etwa stöchiometrischen Mengen eingesetzt, jedoch kann auch ein Überschuß einer der Komponenten, z. B. im Hinblick auf eine möglichst vollständige Umsetzung der anderen Komponente, vorteilhaft sein.

Setzt man gezielt einen Überschuß der Ausgangsstoffe XVII - XVIII ein, mindestens aber 2 Equvivalente pro Mol Ausgangsstoff Ig, so kommt es zu einer Substitution beider Aminowasserstoffe.

### Verfahren D:

Umsetzung eines 1-(Pyridyl-2)-pyrazols der Formel Ig', in der R¹ und R³ bis R⁶ die vorgenannte Bedeutung haben, mit elektrophilen Agenzien der Formel XXI, in der B für eine elektronenziehende Abgangsgruppe steht gemäß DE 35 20 330, JO 2142 - 785 oder EP 201 852:

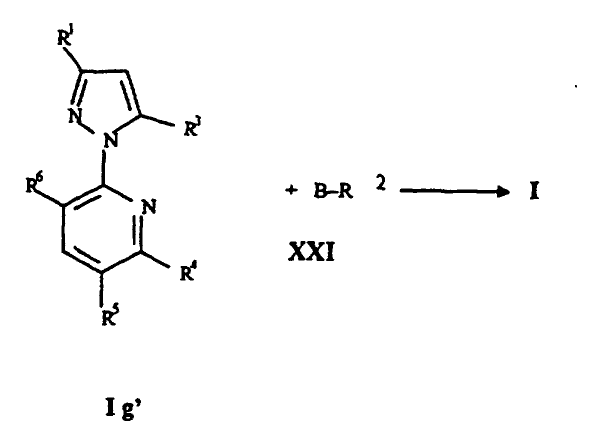

Hierbei steht der Rest R² in der allgemeinen Formel XXI vorzugsweise für Chlor, Brom, Nitro, Rhodano, Formyl, Alkanoyl mit 1 - 4 Kohlenstoffatomen im Alkylteil, C₁-C₄-Alkylsulfonyl, -sulfinyl oder -sulfenyl oder Halogenmethylsulfenyl.

B steht vorzugsweise für Halogen, insbesonderen Chlor oder Brom, für Hydroxy, für Akyl- oder Arylsulfonyloxy, für Alkanoyloxy oder Aroyloxy. Weitere elektrophile Agenzien sind Sulfurylchlorid, Phosphoroxichlorid / Dimethylformamid, Nitriersäure und andere üblicherweise zur elektrophilen Substitution verwendbare Stoffe.

Üblicherweise arbeitet man in einem der vorgenannten inerten Lösungs- oder Verdünnungsmittel.

Die Reaktionstemperatur liegt normalerweise bei 0 - 150°C, vorzugsweise 10 - 110°C.

### Verfahren E:

Diazotierung eines 1-(Pyridyl-2)-5-aminopyrazols der Formel Ih, in der R¹, R² und R⁴ bis R⁶ die vorgenannte Bedeutung besitzen und anschließende Verkochung zu der entsprechenden 5-Hydroxyverbindung nach Houben-Weyl, "Methoden der organischen Chemie" IV. Auflage, Bd. 6/1C, S. 247, Georg Thieme Verlag, Stuttgart 1968, oder Umsetzung zu den entsprechenden Fluorverbindungen ebenda Bd. 5/3, S. 213, Umsetzung unter Sandmeyerbedingungen zu den entsprechenden

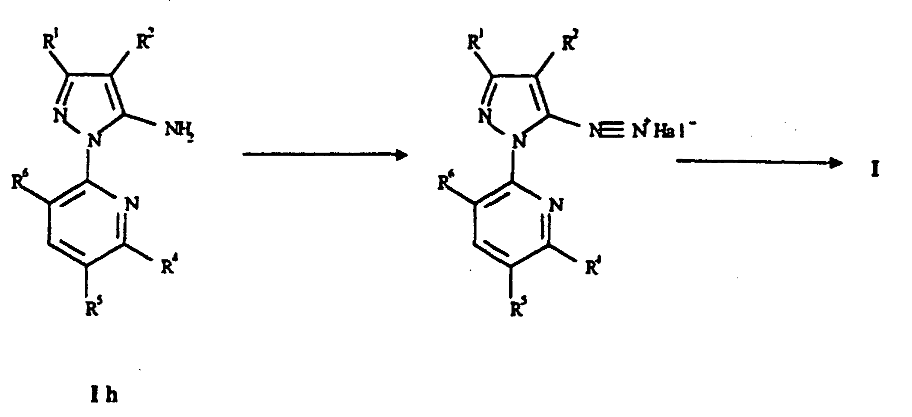

Chlorverbindungen, ebenda Bd. 5/3, S. 846, Bromverbindungen, ebenda Bd. 5/4, S. 437 oder Jodverbindungen, ebenda Bd. 5/4, S. 639, oder Umsetzung zu den entsprechenden Cyanoverbindungen, ebenda Bd. 8, S. 311 oder Rhodanoverbindungen, ebenda Bd. 9, S. 863, oder Umsetzung unter Meerweinbedingungen zu den entsprechenden Sufonsäurechloriden, ebenda Bd. 9, S. 579 und Polgereaktion mit Ammoniak oder Aminen nach Art der Schotten-Baumann-Reaktion. ebenda Bd 9, S. 609 oder Umsetzung mit Mercaptanen zu den entsprechenden Thioethern, ebenda Bd. 9, S. 116 oder mit Alkoholen zu den entsprechendne Ethern, ebenda. Bd. 6/3, S. 81.

### Verfahren F:

Umsetzung eines 1-(Pyridyl-2)-5-halogeno-pyrazols der Formel Ii, in der Halogen, R¹, R², und R⁴ bis R⁶ die vorgenannte Bedeutung besitzen, mit Aminen der Formel XXII, in der R¹⁰ und R¹¹ die 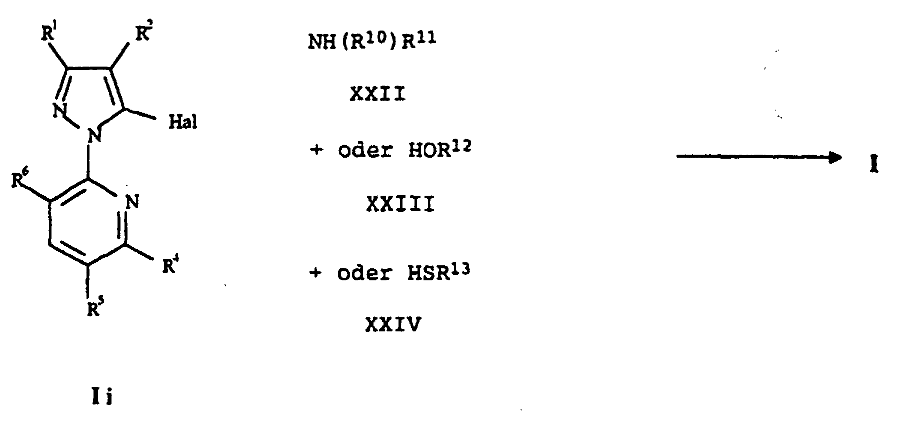 vorgenannten Bedeutungen besitzen, mit Alkoholen der Formel XXIII in der R¹² die vorgenannte Bedeutung besitzt sowie Mercaptanen der Formel XXIV, in der R¹³ die vorgenannte Bedeutung besitzt, nach analogem Verfahren.

Zur Durchführung des Verfahrens setzt man pro Mol 5-Halogenopyrazol Ii im allgemeinen 1,0 - 5,0 mol, vorzugsweise 1,0 - 2,0 mol der Nucleophile XXII - XXIV ein. Die Reaktionsdurchführung, Aufarbeitung und Isolierung der Reaktionsprodukte I erfolgt nach allgemein üblichen Verfahren.

Als Verdünnungsmittel zur Durchführung des Verfahrens F kommen inerte organische Lösungsmittel infrage. Vorzugsweise verwendet man aliphatische oder aromatische, ggf. halogenierte Kohlenwasserstoffe, wie Pentan, Hexan, Heptan, Cyclohexan, Petrolether, Toluol, Xylol, Methylenchlorid, 1,2-Dichlorethan, Chlorbenzol oder Dichlorbenzol, Ether wie Diethylether, Diisopropylether, Methyl-tert.-butylether, Ethylenglykoldimethylether, Tetrahydrofuran oder Dioxan, Ester wie Essigsäureethylester, Nitrile wie Acetonitril oder Propionitril oder Amide wie Dimethylformamid, Dimethylacetamid oder N-Methylpyrrolidon.

Schließlich kann man jedoch auch die als Reaktionskomponenten verwendeten Nucleophile der Formel XXII XXIV in entsprechendem Überschuß gleichzeitig als Verdünnungsmittel einsetzen.

Das Verfahren kann ggf. in Gegenwart eines basischen Katalysators zur Entfernung des entstehenden Halogenwasserstoffs durchgeführt werden. Als solche kommen alle üblichen anorganischen oder organischen Basen in Frage. Vorzugsweise verwendet man die bei Verfahren C genannten Basen.

Man kann jedoch auch direkt die Alkali- oder Erdalkalisalze der Nucleophile XXII - XXIV einsetzen, vorzugsweise die Lithium-, Natrium-, Kalium-, Magnesium- oder Calciumsalze.

Die Temperaturen können bei dem Verfahren F in einem größeren Bereich variiert werden. Im allgemeinen arbeitet man zwischen 0 und 150°C vorzugsweise zwischen 20 und 100°C.

### Verfahren G:

Umsetzung eines 1-(6-Halogeno-pyridyl-2)-pyrazols der Formel Ik, in der R¹ bis R³ und R⁵ und R⁶ die vorgenannte Bedeutung besitzen, mit einem Nucleophil der Formel XXV, in der X Sauerstoff oder Schwefel bedeutet und R¹⁹ die vorgenannte Bedeutung hat:

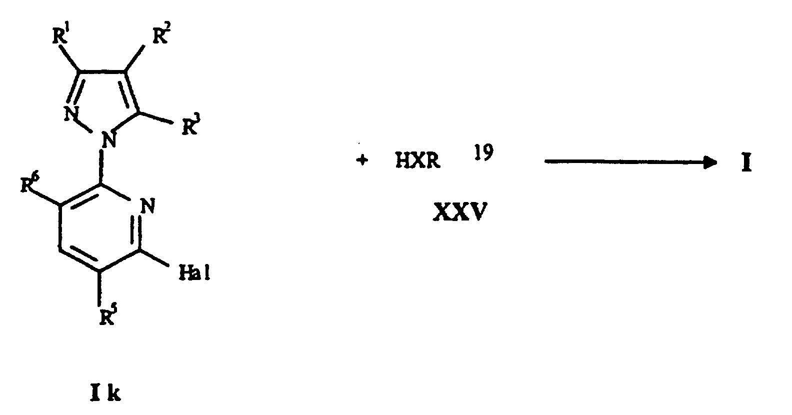

Zur Durchführung des erfindungsgemäßen Verfahrens setzt man pro Mol 1-(6-Halogeno-pyridyl-2)-pyrazol Ik im allgemeinen 0,8 - 2,0 mol, vorzugsweise 0,9 - 1,3 mol des Nucleophils XXV ein.

Als Verdünnungsmittel zur Durchführung des Verfahrens G kommen die bei dem Verfahren F beschriebenen Lösungsmittel infrage.

Man kann jedoch auch das als Reaktionskomponente verwendete Nucleophil der Formel XXV in entsprechendem Überschuß gleichzeitig als Verdünnungsmittel einsetzen.

Das Verfahren kann ggf. in Gegenwart eines basischen Katalysators zur Entfernung des entstehenden Halogenwasserstoffs durchgeführt werden. Als solche kommen alle üblichen anorganischen oder organischen Basen in Betracht. Vorzugsweise verwendet man die bei Verfahren C genannten Basen.

Man kann jedoch auch direkt die Alkali- oder Erdalkalisalze der Nucleophile XXV einsetzen, vorzugsweise die Lithium-, Natrium-, Kalium-, Magnesium- oder Calciumsalze.

Die Temperaturen können bei dem Verfahren G in einem größeren Bereich variiert werden. Im allgemeinen arbeitet man zwischen 0 und 150°C, vorzugsweise zwischen 10 und 80°C.

Die Reaktionsdurchführung, Aufarbeitung und Isolierung der Reaktionsprodukte I erfolgt nach allgemein üblichen Verfahren.

### Verfahren H:

Alternativ zu den bisherigen Verfahren kann der 6-Halogenrest des Pyridyl-2-substituenten in der allgemeinen Formel Ik auch dadurch eingeführt werden, daß man ein l-(Pyridyl-2)-pyrazol Il, in der R¹ bis R³ und R⁵ und R⁶ die vorgenannte Bedeutung besitzen, zuerst mit einem Oxidationsmittel in sein N-Oxid überführt und dann mit einem Phosphoroxihalogenid, beispielsweise Phosphoroxichlorid, zu der 6-Chlor-pyridyl-2-Verbindung Ik' umlagert.

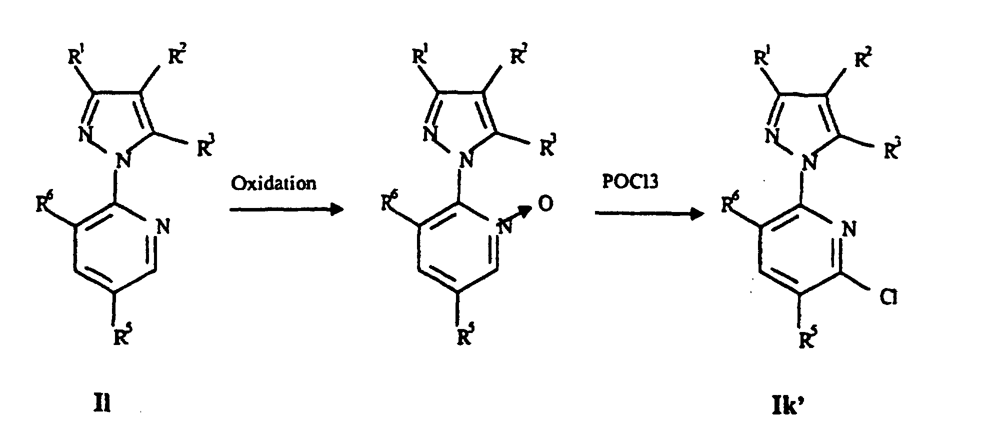

Als Oxidationsmittel verwendet man beispielsweise m-Chlor-perbenzoesäure oder Wasserstoffperoxid in einem inerten Lösungsmittel, beispielsweise einem der vorgenannten Halogenkohlenwasserstoffe wie Methylenchlorid oder einer Alkancarbonsäure wie Eisessig oder Trifluoressigsäure.

Die Oxidation wird im allgemeinen bei einer Temperatur von 0-100°C, vorzugsweise 20 - 60°C durchgeführt und die Umsetzung mit dem Phosphoroxihalogenid bei 80 - 120°C.

Die molaren Verhältnisse, in denen die benötigten Ausgangsverbindungen miteinander umgesetzt werden, betragen im allgeminen 1:0,9 bis 1:1,5 für das Verhältnis von 1-(Pyridyl-2)-pyrazol Il zu dem Oxidationsmittel. Bei dem anschließenden Halogenierungsschritt kann man ein inertes Lösungsmittel wie Chlorbenzol verwenden, zweckmäßig arbeitet man jedoch direkt in überschüssigem Phosphoroxihalogenid als Reaktionsmedium.

Die Konzentration der Edukte im Lösungsmittel(gemisch) beträgt im allgemeinen 0,1 - 5 mol/l, bevorzugt 0,2 - 2 mol/l.

Die einzelnen Verfahrensschritte sind literaturbekannt oder können nach allgemein literaturbekannten Methoden durchgeführt werden (J. org. Chem, Bd. 19, 1633 (1954), EP 422 456).

Sofern nicht anders angegeben, werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

1-(Pyridyl)-pyrazole I mit CH-aciden Substituenten lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium- und Phosphoniumsalze mittels Ammoniak, Phosphonium-, Sulfonium- oder Sulfoxoniumhydroxiden.

Verbindungen I, die eine endständige Aminogruppe tragen, können ferner Säureadditionssalze bilden. Geeignet sind allgemein die Salze von solchen Säuren, welche die herbizide Wirkung von I ebenfalls nicht negativ beeinträchtigen, also z. B. die Hydrochloride und -bromide, Sulfate, Nitrate, Phosphate, Oxalate oder die Dodecylbenzolsulfonate.

### Synthese der Vorprodukte

### 2, 5-Dichlor-6-methoxy-3-trifluormethylpyridin

10,8 (0,06 mol) 30 %ige Natriummethylatlösung wurden innerhalb 20 Minuten unter Rühren bei 0 - 5°C zu 15 g (0,06 mol) 2,3,6-Trichlor-5-trifluormethylpyridin in 200 ml Methyl-tert.-butylether gegeben und 30 Minuten bei gleicher Temperatur nachgerührt. Nach dem Erwärmen auf 20°C wurde der entstandene feine Niederschlag abgesaugt und mit Methyl-tert.-butylether gewaschen. Das organische Filtrat wurde mit Wasser gewaschen, getrocknet und eingeengt, wobei man 14,3 g (97 % d. Th.) der Titelverbindung mit η²⁵_{D} = 1,4890 erhielt.

### 2,5-Dichlor-6-propargyloxy-3-trifluormethylpyridin

9,5 g (0,3952 mol) Natriumhydrid wurden unter Stickstoff spülung in 500 ml Diethylether vorgelegt. Innerhalb 30 Minuten wurden unter Rühren bei 5 - 10°C, 50 ml Propargylalkohol zugeführt und noch 30 Minuten bei 20°C nachgerührt. Nun wurden bei 0 - 5°C unter Rühren innerhalb 20 Minuten 90 g (0,3593 mol) 2,3,6-Trichlor-5-trifluormethylpyridin in 150 ml Diethylether zugeführt, wobei ein feiner Niederschlag entstand. Nach dem Erwärmen auf 20°C wurde wie oben aufgearbeitet, wobei man 96 g (99 % d. Th.) der Titelverbindung mit η_{D}²⁴ = 1,5038 erhielt.

### 3-Chlor-2,6-difluor-5-trifluormethylpyridin

110,3 g (0,44 mol) 2,3,6-Trichlor-5-trifluormethylpyridin und 58,7 g (1,01 mol) Kaliumfluorid wurden unter Rühren zu 500 ml Sulfolan (gemäß S. 42 mit Thionylchlorid getrocknet) gegeben und unter HPLC-Kontrolle 2 1/2 Stunden bei 150 - 160°C gerührt. Nach dem Abkühlen auf 50°C wurden aus dem Reaktionsgemisch 93 g (98 % d. Th.) der Titelverbindung bei 40 - 45°C, 25 mbar abdestilliert. NMR (400 MHz, CDCl₃) 8,2 Pyr-H/t; η_{D}²³ = 1,4229.

### 3-Chlor-6-fluor-2-hydrazino-5-trifluormethylpyridin

10,5 g (0,21 mol) Hydrazinhydrat wurden bei 50°C unter Rühren innerhalb 15 Minuten zu einer Mischung von 21,8 g(0,1 mol) 3-Chlor-2,6-difluor-5-trifluormethylpyridin in 100 ml Propanol gegeben und 2 Stunden bei gleicher Temperatur nachgerührt. Nach dem Abkühlen wurde der Reaktionsansatz auf 1,5 l Wasser gegossen und der ausgefallene Niederschlag abgesaugt. Er wurde zur Reinigung in Essigester aufgenommen und mit Wasser extrahiert. Nach dem Trocknen und Einengen erhielt man 20,9 g (91 % d. Th.) der Titelverbindung von Fp 104 - 106°C; eine sublimierte Probe schmolz bei 111 - 113°C.

### 3-Chlor-6-fluor-2-propargyloxy-5-trifluormethylpyridin:

47,3 g (0,175 mol) 2,5-Dichlor-6-propargyloxy-3-trifluormethylpyridin, 0,5 g (1,89 mmol) 18-Krone-6- und 15,2 g (0,263 mol) Kaliumfluorid wurden zu 150 ml Sulfolan gegeben und 5 Stunden bei 150 - 155°C gerührt. Nach dem Abkühlen wurde das Reaktionsgemisch mit Methyl-tert.-butylether verrührt und von dem anorganischen Niederschlag abgetrennt. Das Filtrat wurde viermal mit Wasser extrahiert, getrocknet und eingeengt. Der Rückstand wurde mit Methylenchlorid über eine Nutsche mit Kieselgel gesaugt und eingeengt, wobei man 31,4 g (71 % d.Th.) der Titelverbindung mit η²⁴_{D} = 1,4732 erhielt.

### 3-Chlor-6-hydrazino-2-methoxy-5-trifluormethylpyridin

10,7 g (0,214 mol) Hydrazinhydrat wurden innerhalb 10 Minuten unter Rühren bei 50°C zu 25,0 g (0,102 mol) 2,5-Dichlor-6-methoxy-3-trifluormethylpyridin in 100 ml Propanol gegeben und 3 Stunden bei 90°C gerührt. Nach dem Abkühlen wurde die entstandene Suspension auf 1,2 Liter Wasser gegossen, der Niederschlag abgesaugt, in Methylenchlorid aufgenommen und mit Wasser gewaschen. Die organische Phase wurde getrocknet, eingeengt und der Rückstand mit Ether / Pentan (1:1) verrührt. Nach dem Absaugen und Trocknen erhielt man 7,8 g (32 % d. Th.) der Titelverbindung vom Fp 140 - 142°C.

### 3-Chlor-6-hydrazino-2-propargyloxy-5-trifluormethylpyridin

Ausgehend von 7,8 (0,155 mol) Hydrazinhydrat und 20 g (0,074 mol) 2,5-Dichlor-6-propargyloxy-3-trifluormethyl-pyridin erhielt man unter den Bedingungen des vorigen Beispiels 11,0 g (56 % d. Th.) der Titelverbindung als farbloses Pulver vom Fp 98 - 102°C.

Nach einem der beschriebenen Verfahren sind weitere Verbindungen IXa oder IXb einschließlich ihrer Vorprodukte XV oder XVI herstellbar bzw. wurden hergestellt:

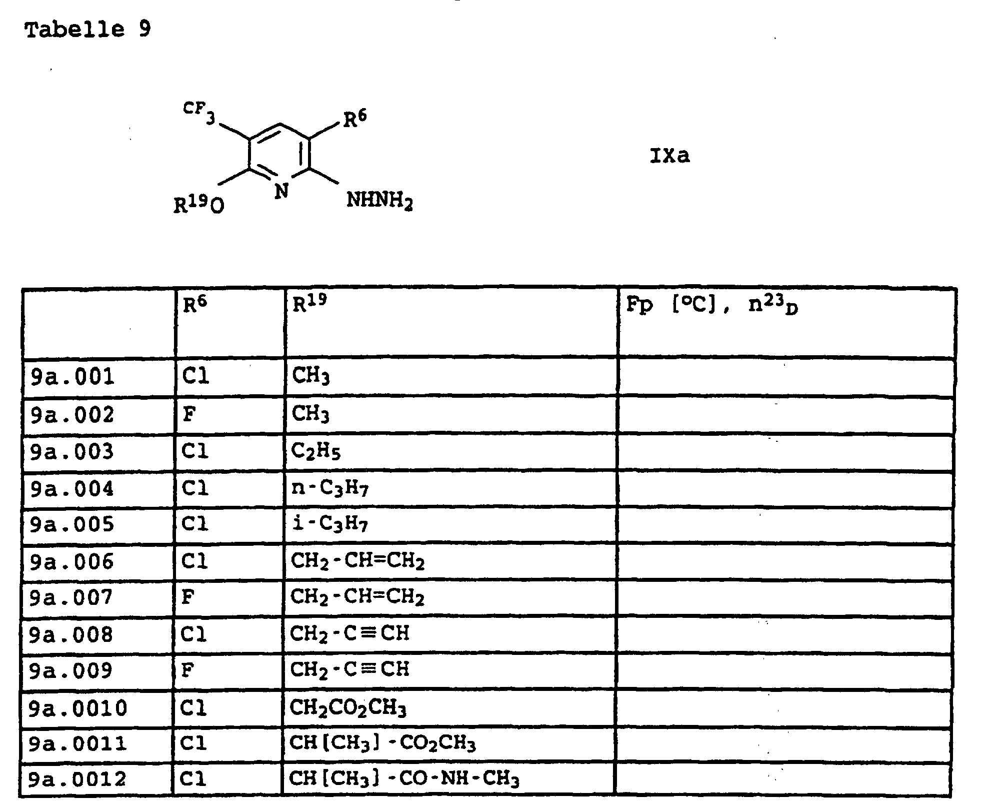

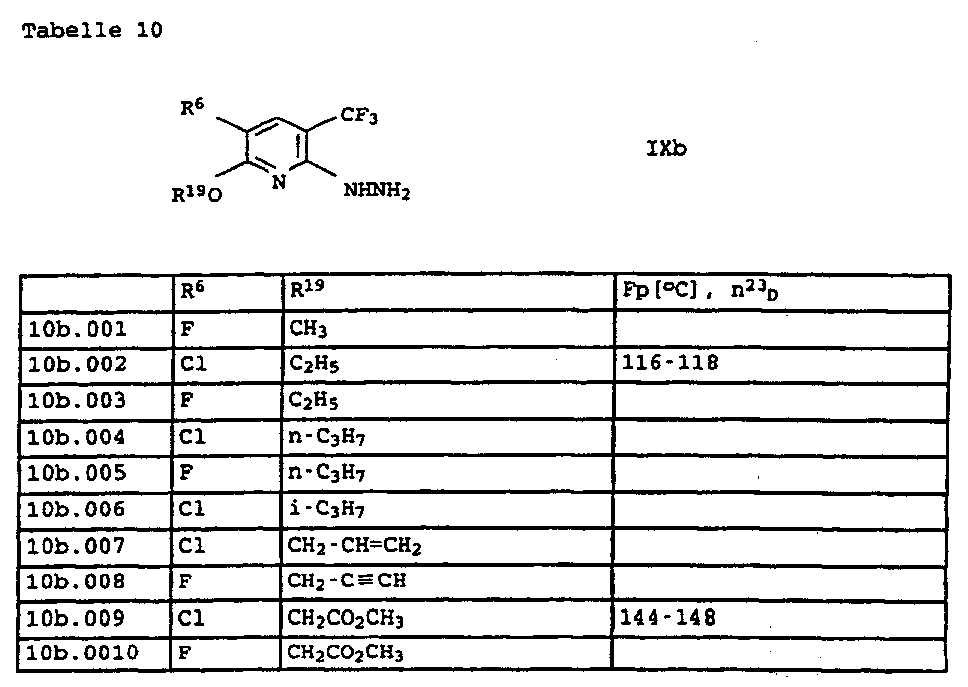

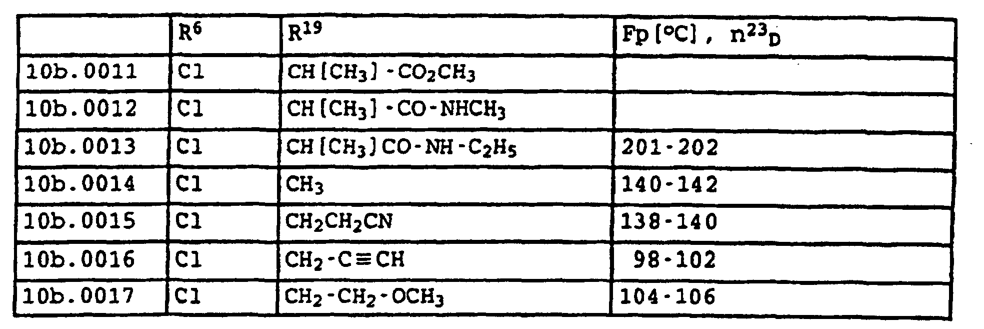

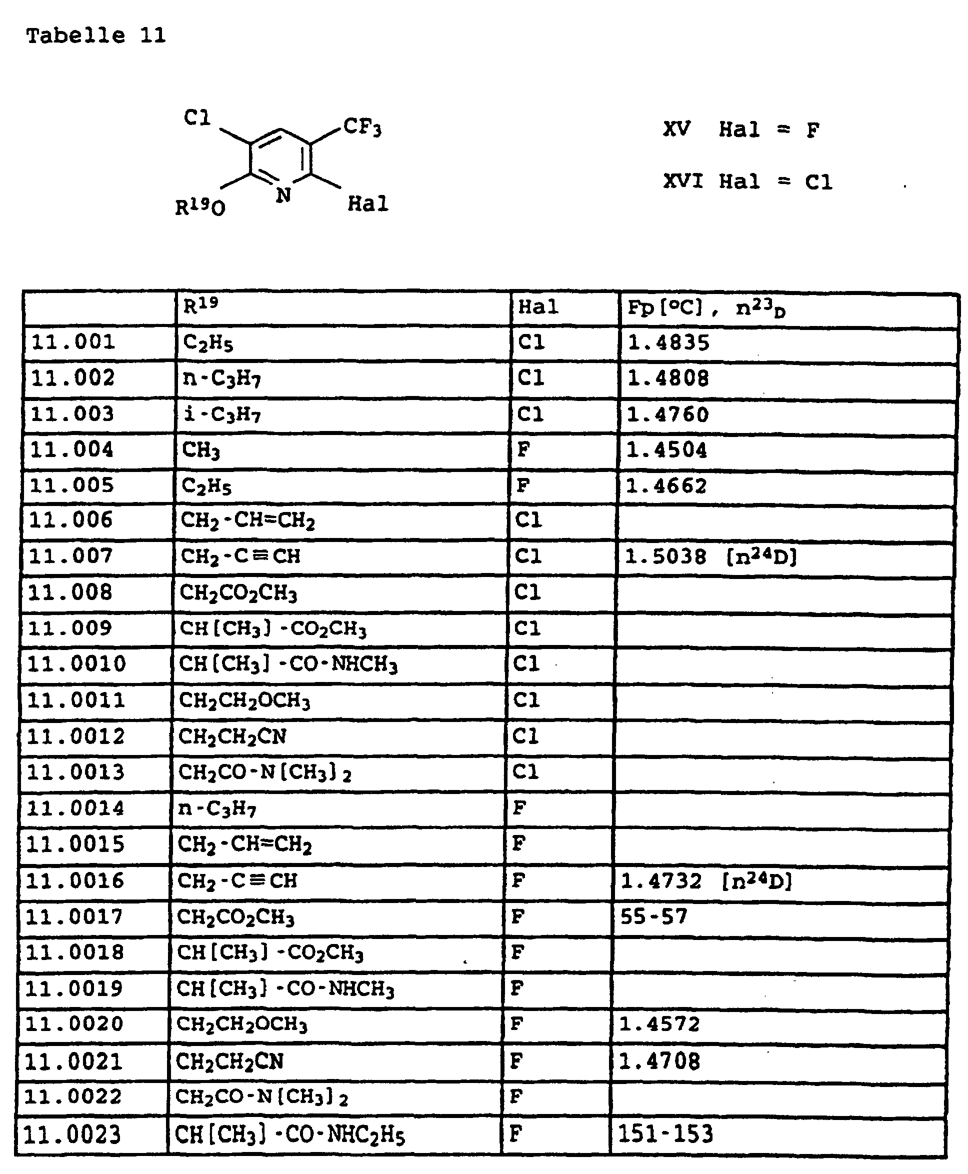

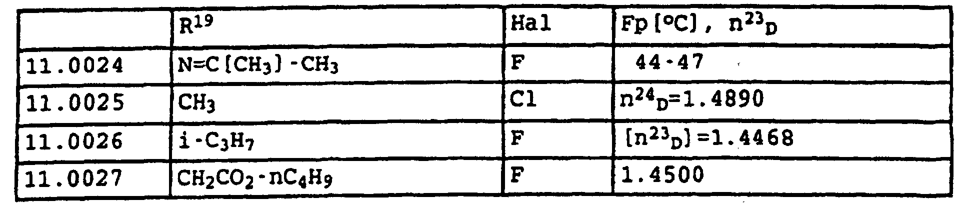

### Beispiel 1:

### 4-Nitro-5-propionamido-1-(3,6-dichlor-5-trifluormethyl-pyridyl-2)-pyrazol

a) 2,8 g (0.0413 mol) 50%iges Wasserstoffperoxid wurden innerhalb 5 Minuten unter Rühren bei 20°C zu einer Mischung von 10 g (0,0275 mol) 4-Nitro-5-propionamido-1-(3-chlor-5-trifluormethylpyridyl-2)-pyrazol und 100 ml Trifluoressigsäure gegeben und 14 Stunden bei 25°C gerührt. Die Reaktionsmischung wurde auf 500 ml Wasser gegossen und 3 x mit Methylenchlorid extrahiert. Die organische Phase wurde nacheinander mit Wasser, Natriumhydrogencarbönat - und gesättigter Kochsalzlösung gewaschen und über Magnesiumsulfat getrocknet. Nach dem Einengen im Vakuum, Verrühren mit Ether / Pentan , Absaugen und Trocknen erhielt man 9,0 g (85 % d. Th.) 4-Nitro-5-propionamido-1-(3-chlor-5-trifluramethyl-(N-oxido)-pyridyl-2)-pyrazol vom Fp 143-146°C.
b) 7,2 g (0,019 mol) N-Oxid aus a) wurden unter Rühren zu 100 ml Phosphoroxichlorid gegeben und 3 Stunden bei 100°C gerührt. Die Reaktionsmischung wurde im Vakuum eingeengt, in Methylenchlorid aufgenommen und zwischen Eiswasser verteilt. Die wäßerige Phase wurde 1 x mit Methylenchlorid extrahiert. Die organischen Auszüge wurden wie oben gewaschen und getrocknet und dann über Aktivkohle und neutrales Aluminiumoxid filtriert. Nach dem Einengen im Vakuum erhielt man 2,6 g (34.3 % d. Th.) der Titelverbindung als zähes Harz. Es kristallisierte nach dem Chromatographieren mit Methylenchlorid über Kieselgel und Einengen mit dem Fp 148 - 154°C (Wirkstoffbeispiel Nr. 9.001).

### Beispiel 2:

### 4-Nitro-5-N(propargyl)-propionamido-1-(3,6-dichlor-5-trifluormethyl-pyridyl-2)-pyrazol

3,2 g (0,008 mol) der Verbindung aus dem vorstehenden Beispiel in 50 ml Dimethylformamid wurden innerhalb 10 Minuten unter Rühren zu einer Mischung von 0,22 g (0,0088 mol) 95 %igem Natriumhydrid in 50 ml Dimethylformamid gegeben. Es wurde 30 Minuten bei 50°C gerührt und dann bei 80°C 1,05 g (0.0088 mol) 3-Brompropin innerhalb 3 Minuten zugegeben. Nach 8 Stunden Rühren bei 80°C wurde abgekühlt und im Vakuum eingeengt. Der Rückstand wurde zwischen Wasser / Methylenchlorid verteilt, die organische Phase über Magnesiumsulfat getrocknet, über Kieselgel filtriert und eingeengt. Nach dem Anrühren in Ether / Pentan erhielt man 2,7 g (77,4 % d. Th.) der Titelverbindung vom Fp 107 - 110°C (Wirkstoffbeispiel Nr. 9.006).

### Beispiel 6:

### 5-Amino-1-(5-chlor-6-propargyloxy-3-trifluormethyl-pyridyl-2)-pyrazol-4-carbonsäurethylester

19,1 g (0,113 mol) Ethoxymethylen-cyanessigester wurden innerhalb 10 Minuten unter Rühren zu einer Mischung von 30 g (0,113 mol) 5-Chlor-6-propargyloxy-3-trifluormethyl-pyridyl-2-hydrazin und 300 ml Methylglykol gegeben und 30 Minuten bei 80°C gerührt. Nach weiteren 4 Stunden Rühren bei 120°C wurde das Reaktionsgemisch im Vakuum eingeengt und in Ether / Pentan verrührt. Nach dem Absaugen erhielt man 36,1 g (82,2 % d. Th.) der Titelverbindung vom Fp 118 - 119°C (Wirkstoffbeispiel Nr. 9.005).

### Beispiel 7:

### 5-Allylamino-4-nitro-1-(3-chlor-5-trifluormethyl-pyridyl-2)-pyrazol

1,0 g (0,0082 mol) 3-Brompropen wurden unter Rühren zu einer Mischung von 2,3 g (0,0075 mol) 5-Amino-4-nitro-1-(3-chlor-5-trifluormethyl-pyridyl-2)-pyrazol, 0,6 g (0,0041 mol) Kaliumcarbonat und 50 ml Dimethylformamid gegeben und 2 Stunden bei 100°C gerührt. Nach dem Abkühlen wurde im Vakuum eingeengt und der Rückstand zwischen Wasser und Methylenchlorid verteilt. Die organische Phase wurde getrocknet, über Kieselgel abgesaugt und eingeengt, wobei man 1,6 g (61,3 % d. Th.) der Titelverbindung als zähes Harz erhielt. Nach dem Chromatographieren über Kieselgel und Einengen erhielt man farblose Kristalle vom Fp 94 - 95°C (Wirkstoffbeispiel Nr. 9.003).

### Beispiel 8:

### 5-Amino-4-rhodano-1-[3-chlor-6-fluor-5-trifluormethyl-pyridyl-2]-pyrazol

3,3 g (0,0335 mol) Kaliumthiocyanat wurden innerhalb 2 Minuten unter Rühren bei 10°C zu einer Mischung von 4,7 g (0,0168 mol) 5-Amino-[3-chlor-6-fluor-5-trifluormethylpyridyl-2]-pyrazol und 70 ml konz. Essigsäure gegeben. Anschließend wurden innerhalb 5 Minuten 2,7 g Brom in 10 ml Eisessig zugefügt und noch 15 Minuten bei 23°C gerührt. Die Reaktionsmischung wurde auf 300 ml Eiswasser gegeben und der ausgefallene Niederschlag abgesaugt, in Methylenchlorid aufgenommen und über Magnesiumsulfat getrocknet. Nach dem Absaugen über Kieselgel, Einengen im Vakuum, Verrühren mit Ether/Petrolether 1:1, Absaugen und Trocknen erhielt man 4,2 g (74 % d.Th.) der Titelverbindung vom Fp 109-114°C [Wirkstoffbeispiel Nr. 6a.001].

### Beispiel 10

### 5-Allylamino-4-nitro-1-[3-fluor-5-trifluormethyl-pyridyl-2]-pyrazol

0,5 g [0,0085 mol] Allylamin wurden innerhalb 2 Minuten unter Rühren bei 22°C zu 1,5 g [0,0042 mol] 5-Brom-4-nitro-1-[3-fluor-5-trifluormethyl-pyridyl-2]-pyrazol in 50 ml Methyl-tert.-butylether gegeben und dann 2 Stunden bei 50°C gerührt. Nach dem Abkühlen wurde mit 100 ml Essigester verdünnt, mit 0,1 n Salzsäure extrahiert, mit wasser und mit Kochsalzlösung gewaschen, getrocknet und eingeengt. Nach dem Verrühren mit Pentan erhielt man 1,1 g der Titelverbindung vom Fp. 90-92°C [Wirkstoffbeispiel Nr. 9.034].

In den folgenden Tabellen sind weitere Verbindungen I aufgeführt. die analog den Beispielen hergestellt wurden oder entweder nach den vorstehend beschriebenen Verfahren oder nach an sich bekannten Methoden herstellbar sind.

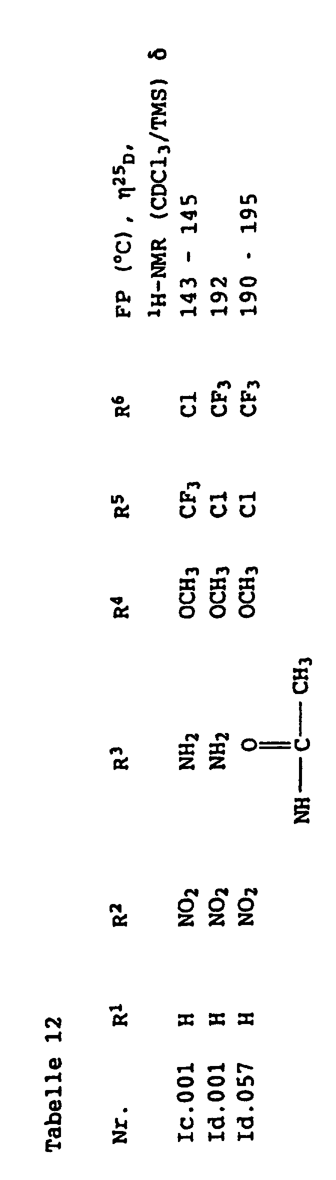

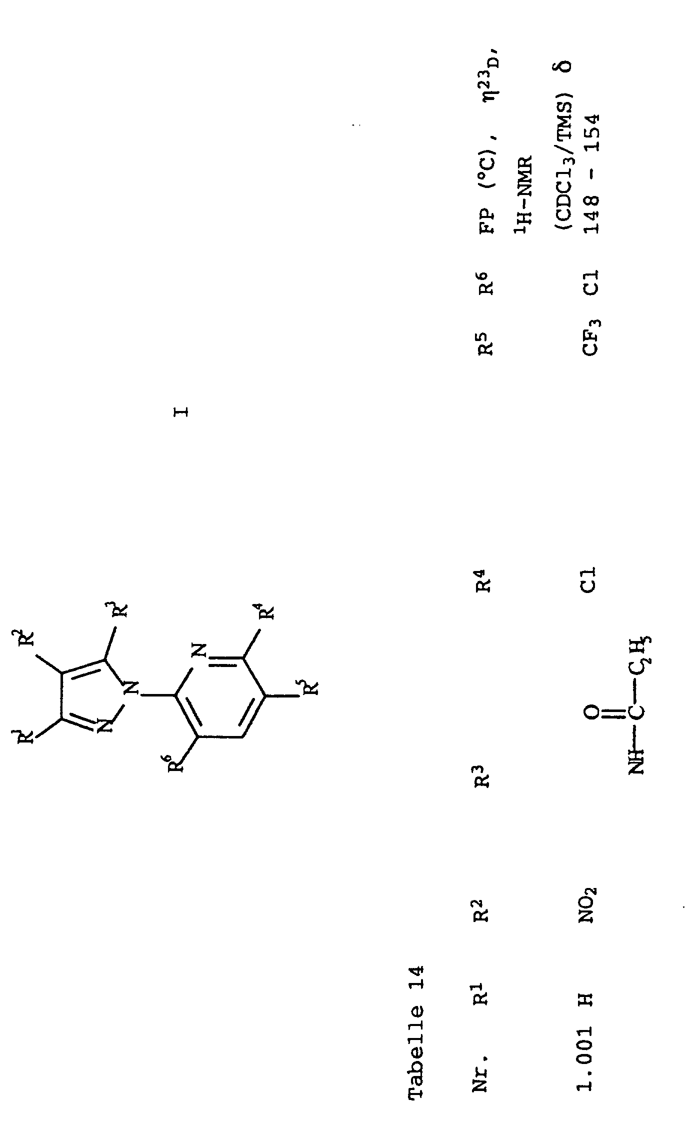

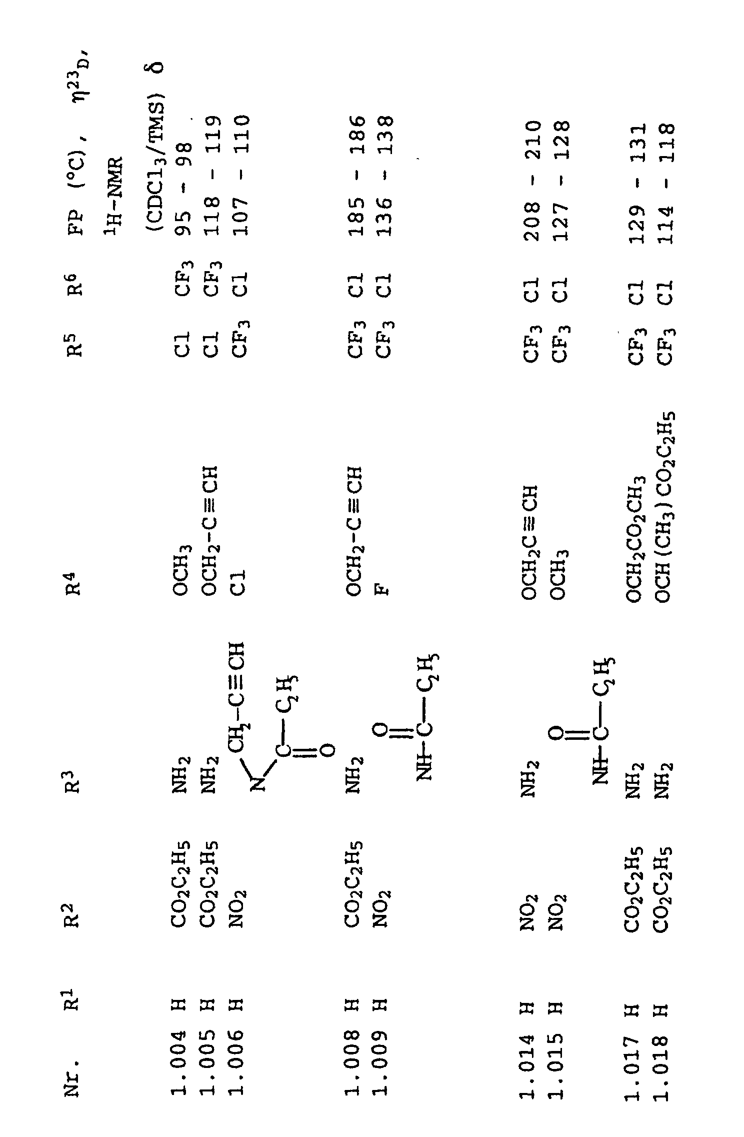

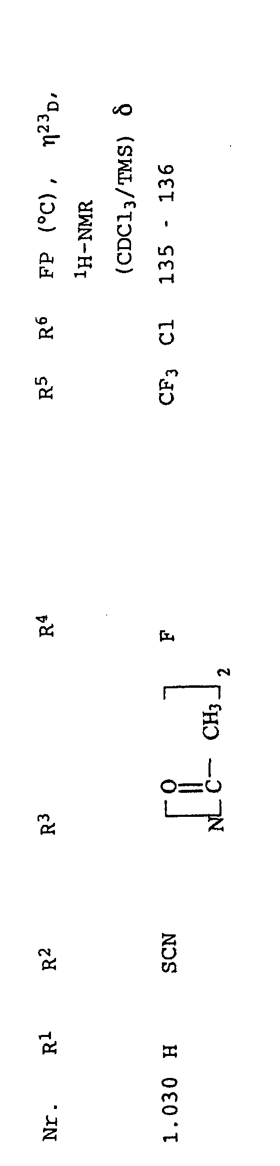

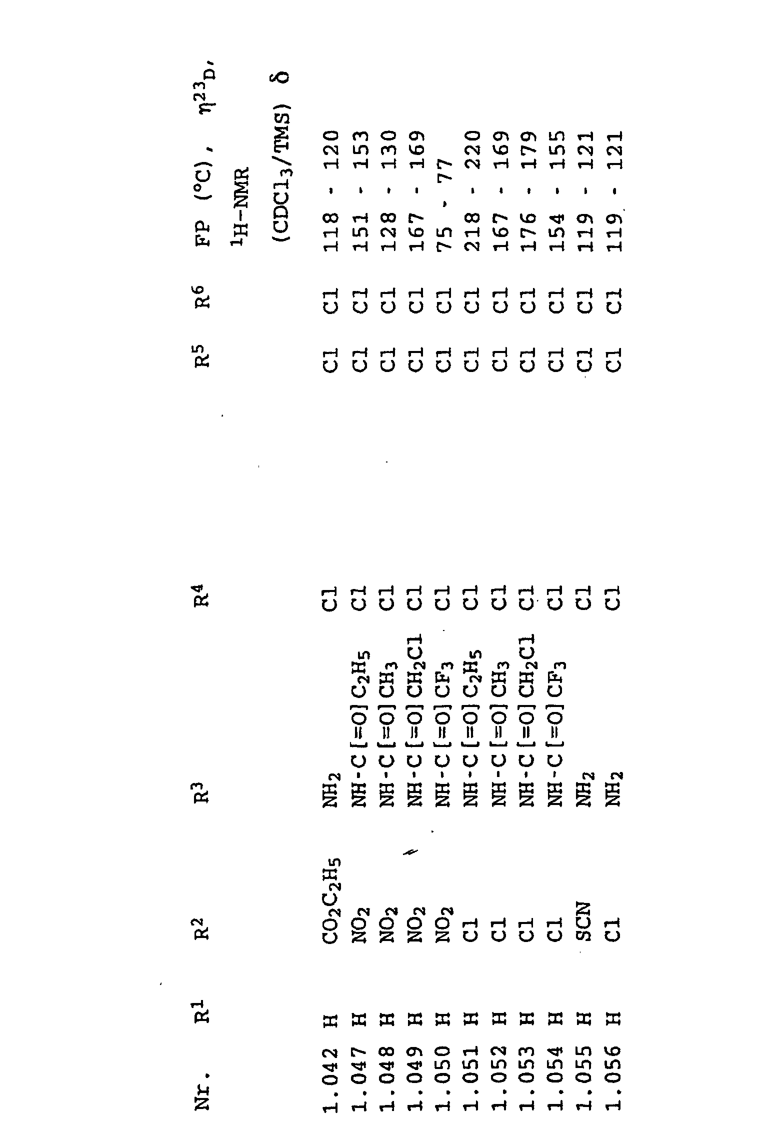

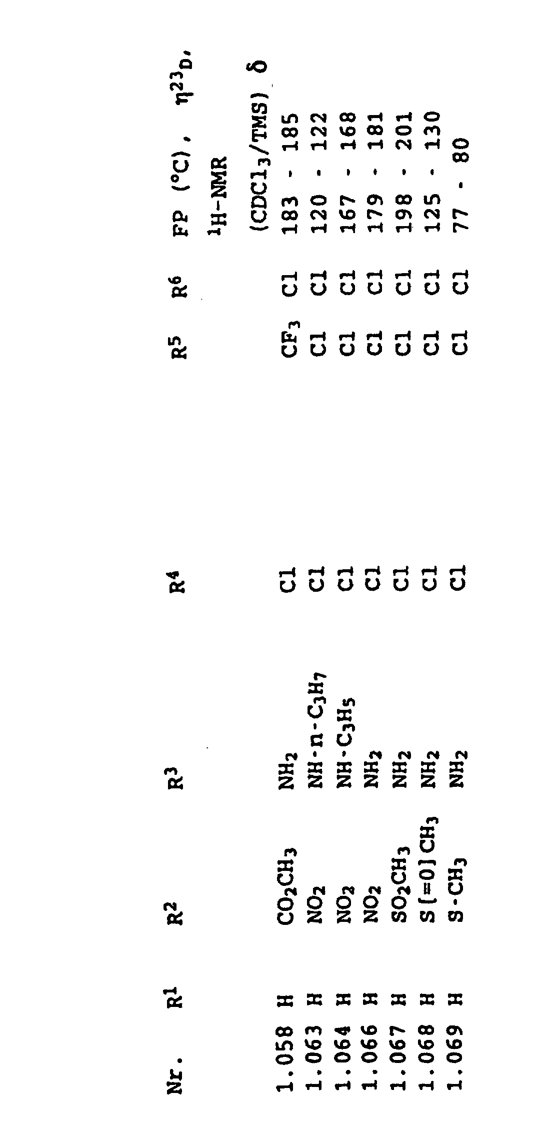

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 1.001 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II. 20 Gewichtsteile der Verbindung Nr. 1.005 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 1.006 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 1.007 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 1.008 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 1.009 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 1.010 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 1.011 wird in einer Mischung gelöst, die aus 80 Gewichtsteilen Cyclohexanon und 20 Gewichtsteilen Emulphor EL (ethoxyliertes Rizinusöl/-casteroil) besteht. Man erhält ein stabiles Emulsionskonzentrat.

Zur Verbreiterung des Wirkungsspektrums und zur Erzielung synergistischer Effekte können die 1-(Pyridyl)-pyrazole I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxyx- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

### Anwendungsbeispiele

Die herbizide Wirkung der 1-(Pyridyl)pyrazole der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 0.0312 bzw. 0.0156 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Abutilon theophrasti | Chinesischer Hanf | velvet leaf |
| Oryza sativa | Reis | rice |
| Solanum nigrum | Schwarzer Nachtschatten | black nightshade |

## Patentansprüche

1. 1-(Pyridyl)-pyrazole der allgemeinen Formel I 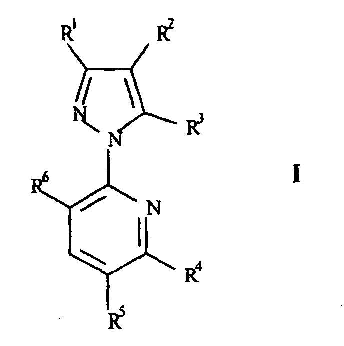 in der die Substituenten die folgende Bedeutung haben:
R¹ Wasserstoff;
R² C₁-C₄-Alkoxy, C₁-C₄-Halogenalkoxy, NO₂, Halogen, Rhodano, Amino, C(=O)R⁷, S(O)ₙR⁸ oder NH-C(=O)R⁹;
R³ Amino, Halogen, Rhodano, Cyano, Nitro, Hydroxy, C₁-C₃-Alkyl, C₁-C₃-Halogenalkyl, S(O)ₙR⁸, N(R¹⁰)R¹¹, OR¹², SR¹³, N=C(R¹⁴)-N(R¹⁵)R¹⁶ oder N=C(R¹⁷)R¹⁸;
R⁴ Halogen, einen Rest XR¹⁹;
R⁵ und R⁶ unabhängig voneinander Halogen oder Trifluormethyl;
R⁷ C₁-C₄-Alkyl, C₁-C₄-Halogenalkyl, C₁-C₄-Alkoxy oder C₁-C₄-Alkylamino;
R⁸ C₁-C₄-Alkyl, Trifluormethyl, C₁-C₄-Alkoxy, Chlor, Amino, Methylamino oder Ethylamino;
R⁹ C₁-C₄-Alkyl, C₁-C₄-Halogenalkyl oder C₁-C₄-Alkoxy;
R¹⁰ Wasserstoff, C₁-C₄-Alkyl oder C(=O)R⁷;
R¹¹ C₁-C₄-Alkyl, C₃-C₄-Alkenyl, C₃-C₄-Alkinyl, C(=O)R⁷ oder S(O)ₙR⁸;
R¹² C₁-C₃-Alkyl, C₁-C₃-Halogenalkyl, C₃-C₄-Alkenyl, C₃-C₄-Alkinyl oder C₁-C₅-Alkoxycarbonyl-C₁-C₄-alkyl;
R¹³ C₁-C₄-Alkoxycarbonyl-C₁-C₄-alkyl;
R^{14,16,17} Wasserstoff oder C₁-C₃-Alkyl;
R¹⁵ C₁-C₄-Alkyl;
R¹⁸ C₁-C₄-Alkyl, C₁-C₄-Halogenalkyl oder C₂-C₄-Alkenyl;
R¹⁹ C₁-C₄-Alkyl, C₃-C₄-Alkenyl, C₃-C₄-Alkinyl, C₁-C₄-Halogenalkyl, C₁-C₄-Cyanoalkyl, C₁-C₄-Alkoxycarbonyl-C₁-C₄-alkyl, im Falle von X = O zusätzlich C₁-C₄-Alkylamino oder C₁-C₄-Alkylidenimino;
X Sauerstoff oder Schwefel;
Halogen Fluor oder Chlor und
n 0, 1 oder 2 bedeuten,
sowie die N-Oxide und die landwirtschaftlich brauchbaren Salze der Verbindungen I.

2. Verwendung von 1-(Pyridyl)-pyrazolen der Formel I gemäß Anspruch 1, als Herbizide.

3. Herbizides Mittel, enthaltend eine herbizid wirksame Menge mindestens eines 1-(Pyridyl)-pyrazols der Formel 1 oder ein landwirtschaftlich brauchbares Salz von 1, gemäß Anspruch 1, und mindestens einen inerten flüssigen und / oder festen Trägerstoff sowie gewünschtenfalls mindestens ein Adjuvants.

4. Verfahren zur Herstellung von herbizid wirksamen Mitteln, **dadurch gekennzeichnet, daß** man eine herbizid wirksame Menge mindestens eines 1-(Pyridyl)-pyrazols der Formel 1 oder ein landwirtschaftlich brauchbares Salz von 1 gemäß Anspruch 1, und mindestens einen inerten flüssigen und / oder festen Trägerstoff sowie gewünschtenfalls mindestens ein Adjuvants mischt.

5. Verfahren zur Herstellung von desiccant und / oder defoliant wirksamen Mitteln, **dadurch gekennzeichnet, daß** man eine desiccant und / oder defoliant wirksame Menge mindestens eines 1-(Pyridyl)-pyrazols der Formel I oder ein landwirtschaftlich brauchbares Salz von 1, gemäß Anspruch 1, und mindestens einen inerten flüssigen und / oder festen Trägerstoff sowie gewünschtenfalls mindestens ein Adjuvants mischt.

6. Verfahren zur Bekämpfung von unerwünschtem Pflanzenwuchs, **dadurch gekennzeichnet, daß** man eine herbizid wirksame Menge mindestens eines substituierten 1-(Pyridyl)-pyrazols der Formel 1 oder eines landwirtschaftlich brauchbaren Salzes von 1, gemäß Anspruch 1, auf Pflanzen, deren Lebensraum oder auf Saatgut einwirken läßt.

## Claims

1. A 1-(pyridyl)pyrazole of the general formula I 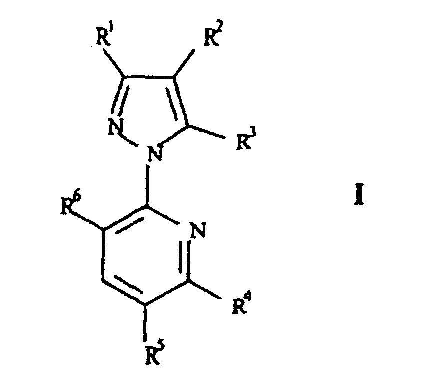 in which the substituents have the following meaning:
R¹ is hydrogen;
R² is C₁-C₄-alkoxy, C₁-C₄-haloalkoxy, NO₂, halogen, thiocyanato, amino, C(=O)R⁷, S(O)ₙR⁸ or NH-C(=O)R⁹;
R³ is amino, halogen, thiocyanato, cyano, nitro, hydroxyl, C₁-C₃-alkyl, C₁-C₃-halo-alkyl, S(O)ₙR⁸, N(R¹⁰)R¹¹, OR¹², SR¹³, N=C(R¹⁴)-N(R¹⁵)R¹⁶ or N=C(R¹⁷)R¹⁸;
R⁴ is halogen, a radical XR¹⁹;
R⁵ and R⁶ independently of one another are halogen or trifluoromethyl;
R⁷ is C₁-C₄-alkyl, C₁-C₄-haloalkyl, C₁-C₄-alkoxy or C₁-C₄-alkylamino;
R⁸ is C₁-C₄-alkyl, trifluoromethyl, C₁-C₄-alkoxy, chlorine, amino, methylamino or ethylamino;
R⁹ is C₁-C₄-alkyl, C₁-C₄-haloalkyl or C₁-C₄-alkoxy;
R¹⁰ is hydrogen, C₁-C₄-alkyl or C(=O)R⁷;
R¹¹ is C₁-C₄-alkyl, C₃-C₄-alkenyl, C₃-C₄-alkynyl, C(=O)R⁷ or S(O)ₙR⁸;
R¹² is C₁-C₃-alkyl, C₁-C₃-haloalkyl, C₃-C₄-alkenyl, C₃-C₄-alkynyl or C₁-C₅-alkoxycarbonyl-C₁-C₄-alkyl;
R¹³ is C₁-C₄-alkoxycarbonyl-C₁-C₄-alkyl;
R^{14,16,17} is hydrogen or C₁-C₃-alkyl;
R¹⁵ is C₁-C₄-alkyl;
R¹⁸ is C₁-C₄-alkyl, C₁-C₄-haloalkyl or C₂-C₄-alkenyl;
R¹⁹ is C₁-C₄-alkyl, C₃-C₄-alkenyl, C₃-C₄-alkynyl, C₁-C₄-haloalkyl, C₁-C₄-cyanoalkyl, C₁-C₄-alkoxycarbonyl-C₁-C₄-alkyl, if X = O additionally C₁-C₄-alkylamino or C₁-C₄-alkylideneimino;
X is oxygen or sulfur;
halogen is fluorine or chlorine and
n is 0, 1 or 2,
and the N-oxides and the agriculturally utilizable salts of the compounds I.

2. The use of 1-(pyridyl)pyrazoles of the formula I, as claimed in claim 1, as herbicides.

3. A herbicidal composition, comprising a herbicidally active amount of at least one 1-(pyridyl)pyrazole of the formula I or an agriculturally utilizable salt of I, as claimed in claim 1, and at least one inert liquid and/or solid carrier and, if desired, at least one adjuvant.

4. A process for the preparation of herbicidally active compositions, which comprises mixing a herbicidally active amount of at least one 1-(pyridyl)pyrazole of the formula I or an agriculturally utilizable salt of I as claimed in claim 1, and at least one inert liquid and/or solid carrier and, if desired, at least one adjuvant.

5. A process for the preparation of compositions having desiccant and/or defoliant activity, which comprises mixing an amount of at least one 1-(pyridyl)pyrazole of the formula I, having desiccant and/or defoliant activity, or an agriculturally utilizable salt of I, as claimed in claim 1, and at least one inert liquid and/or solid carrier and, if desired, at least one adjuvant.

6. A process for the control of undesired plant growth, which comprises allowing a herbicidally active amount of at least one substituted 1-(pyridyl)pyrazole of the formula I or of an agriculturally utilizable salt of I, as claimed in claim 1, to act on plants, their habitat or on seed.

## Revendications

1. 1-(pyridyl)-pyrazole de formule générale I 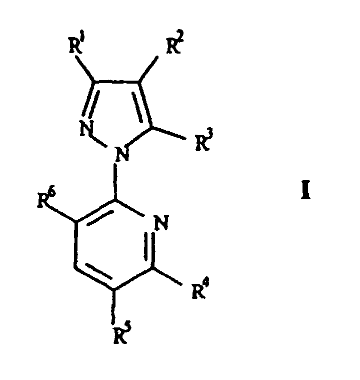 dans laquelle les substituants ont la signification suivante :
R¹ représente un atome d'hydrogène ;
R² représente un groupe alcoxy en C₁ à C₄, halogénoalcoxy en C₁ à C₄, NO₂, halogéno, thiocyanate, amino, C(=O)R⁷, S(O)ₙR⁸ ou NH-C(=O)R⁹ ;
R³ représente un groupe amino, halogéno, thiocyanate, cyano, nitro, hydroxy, alkyle en C₁ à C₃, halogénoalkyle en C₁ à C₃, S(O)ₙR⁸, N(R¹⁰)R¹¹, OR¹², SR¹³, N=C(R¹⁴)-N(R¹⁵)R¹⁶ ou N=C(R¹⁷)R¹⁸ ;
R⁴ représente un atome d'halogène, un groupe XR¹⁹ ;
R⁵ et R⁶ représentent, indépendamment l'un de l'autre, un atome d'halogène ou un groupe trifluorométhyle ;
R7 représente un groupe alkyle en C₁ à C₄, halogénoalkyle en C₁ à C₄, alcoxy en C₁ à C₄ ou alkylamino en C₁ à C₄ ;
R⁸ représente un groupe alkyle en C₁ à C₄, trifluorométhyle, alcoxy en C₁ à C₄, un atome de chlore, un groupe amino, méthylamino ou éthylamino ;
R⁹ représente un groupe alkyle en C₁ à C₄, halogénoalkyle en C₁ à C₄ ou alcoxy en C₁ à C₄ ;
R¹⁰ représente un atome d'hydrogène, un groupe alkyle en C₁ à C₄ ou C(=O)R⁷
R¹¹ représente un groupe alkyle en C₁ à C₄, alcényle en C₃ à C₄, alcynyle en C₃ à C₄, C(=O)R⁷ ou S(O)ₙR⁸ ;
R¹² représente un groupe alkyle en C₁ à C₃, halogénoalkyle en C₁ à C₃, alcényle en C₃ à C₄, alcynyle en C₃ à C₄, ou (alcoxy en C₁ à C₅)carbonyl-(alkyle en C₁ à C₄) ;
R¹³ représente un groupe (alcoxy en C₁ à C₅)carbonyle-(alkyle en C₁ à C₄) ;
R¹⁴, R¹⁶, R¹⁷ représentent un atome d'hydrogène ou un groupe alkyle en C₁ à C₄,
R¹⁵ représente un groupe alkyle en C₁ à C₄ ;
R¹⁸ représente un groupe alkyle en C₁ à C₄, halogénoalkyle en C₁ à C₄ ou alcényle en C₂ à C₄ ;
R¹⁹ représente un groupe alkyle en C₁ à C₄, alcényle en C₃ à C₄, alcynyle en C₃ à C₄, halogénoalkyle en C₁ à C₄, cyanoalkyle en C₁ à C₄, (alcoxy en C₁ à C₅)carbonyl-(alkyle en C₁ à C₄), et si X=0, un groupe (alkyle en C₁ à C₄)amino ou (alkyle en C₁ à C₄)idénimino ;
X représente un atome d'oxygène ou de soufre ; un halogène fluor ou chlore et
n vaut 0, 1 ou 2,
les N-oxydes et les sels des composés I utilisables en agriculture.

2. Utilisation des 1-(pyridyl)-pyrazoles de formule générale I selon la revendication 1, en tant qu'herbicides.

3. Agent herbicide, contenant une quantité efficace sur le plan herbicide d'au moins un 1-(pyridyl)-pyrazole de formule I ou un sel de I utilisable en agriculture, selon la revendication 1, et au moins un support inerte liquide et/ou solide, ainsi que, si souhaité, au moins un adjuvant.

4. Procédé pour préparer un agent herbicide efficace, **caractérisé en ce que** l'on mélange une quantité efficace sur le plan herbicide d'au moins un 1-(pyridyl)-pyrazole de formule I ou un sel de I utilisable en agriculture selon la revendication 1, et au moins un support inerte liquide et/ou solide, ainsi que, si souhaité, au moins un adjuvant.

5. Procédé pour préparer un agent efficace de dessiccation et/ou de défoliation, **caractérisé en ce que** l'on mélange une quantité efficace dessiccante et/ou défoliante d'au moins un 1-(pyridyl)-pyrazole de formule I ou un sel de I utilisable en agriculture, selon la revendication 1, et au moins un support inerte liquide et/ou solide, ainsi que, si souhaité, au moins un adjuvant.

6. Procédé pour combattre la croissance de plantes indésirables, **caractérisé en ce qu'**on laisse agir une quantité efficace sur le plan herbicide d'au moins un 1-(pyridyl)-pyrazole de formule I substitué ou d'un sel de I utilisable en agriculture, selon la revendication 1, sur des plantes, sur leur environnement ou leurs semences.
